# EUROPEAN PATENT APPLICATION

(11) **EP 4 201 307 A1**
(43) Date of publication of application: **28.06.2023**
(21) Application number: 21383224.9
(22) Date of filing: 27.12.2021
(51) Int. Cl.: A61B 5/00

(54) **SYSTEM FOR PROVIDING FAILED BACK SURGERY SYNDROME ASSESSMENT DATA**

(71) Applicant: Fundacion Tecnalia Research and Innovation, 20009 Donostia (ES); Fundación para la Investigación Biomédica del Hospital Universitario Ramón y Cajal, 28034 Madrid (ES)
(72) Inventor: IMATZ OJANGUREN, Eukene, E-20009 DONOSTIA (ES); DE LA CALLE REVIRIEGO, José Luis, 28034 Madrid (ES); KELLER, Thierry, E-20009 DONOSTIA (ES); ZAMORA ROMERO, Javier, 28034 Madrid (ES); OTAZUA URIBE, Enaitz, E-20009 DONOSTIA (ES)
(74) Representative: Hoffmann Eitle

(57) **Abstract**

A system for Current Perception Threshold quantification of a user. The system comprises: one or more electrode pairs (112); a stimulator (12) configured to provide electrical stimuli between the pair of electrodes (1120, 1125) of each of the one or more electrode pairs (112); a feedback element (16) configured to determine whether the electrical stimuli is felt by the user; and a processor (14), wherein the processor is configured to control the stimulator (12) and receive an indication from the feedback element (16) whether the electrical stimuli is felt by the user. The one or more electrode pairs (122) are provided on a substrate for placing the electrode pairs (112) to the skin of the user, and the system is further configured to trigger the electrical stimuli of the buttock, the lumbar, the thigh and/or the calf skin areas of the user through the one or more of electrode pairs (112), wherein each electrode pair (112) defines a sub-area within the area. The processor (14) is further configured to: i. for each frequency of the set of frequencies for each of a plurality of electrode pair positions, to receive an indication of the location of the electrode pair positions on the buttock, the lumbar, the thigh and/or the calf, and control the stimulator (12) to provide two or more electrical stimuli at the frequency and electrode pair positions, the two or more electrical stimuli having different electrical intensities; ii. for each electrical stimulus, receive an indication by the feedback element (16) whether the electrical stimuli was felt by the user; iii. for each frequency at each electrode pair position, determine a threshold value for the lowest electrical intensity felt by the user based on the received indications for each electrical stimulus; and iv. for each frequency for the buttock, the lumbar, the thigh and/or the calf, calculate an average threshold value indicative of the lowest electrical intensity felt by the user, based at least in part on the threshold values determined in iii).

## Description

### TECHNICAL FIELD OF THE INVENTION

The present invention pertains to the field of medicine and particularly to the field of failed back surgery syndrome. The invention relates to a device, a computer-implemented method and a computer program to generate data for classifying patients with failed back surgery syndrome (FBSS) eligible for spinal cord stimulation (SCS) treatment.

### BACKGROUND

Failed Back Surgery Syndrome (FBSS) is a complex clinical frame involving persistent lower back and/or leg pain often associated with a combined neuropathic and nociceptive nature after a technically successful spine surgery. Neuropathic pain is related to damage affecting the somatosensory nervous system, while nociceptive pain is related to the activation of cells specialised to detect noxious stimuli. That is, although the spine surgery might be successful with regard to the problem being faced, the surgery can provoke changes in the nerve configuration, or increased sensitivity that lead to associated pain in the lower back and/or leg.

It is important to properly identify if the pain is neuropathic as its treatment differs to the nociceptive pain. However, neuropathic pain maybe difficult to diagnose and differentiate from other types of pain. For most FBSS patients suffering pain, no precise organic cause can be identified; therefore, a rigorous assessment is needed to characterize co-existing neuropathic features and guide a treatment plan. One of the most common treatments is the Spinal Cord Stimulation (SCS), which has shown success in cases of neuropathic back pain FBSS. SCS treatment comprises the stimulation of precise nerves when false pain triggering event are detected, so that the pain signals are at least diminished when reaching the central nervous system (CNS). Therefore, SCS treatment is specifically useful to patients with neuropathic pains, and there must be a rigorous study of each patient's case receiving SCS treatment. Currently self-reported questionnaires and pain descriptions are used for assessing pain, which are subjective measures that can result in lack of accuracy. Some studies have shown that 20% of people that undergo surgery after checking their suitability with an SCS implant by means of pain questionnaires, result non-responsive to the treatment during the test phase. Other studies describe failure rates of about 60% in patients subjected to trial periods and of about 25-40% in permanent implants at long term in patients with FBSS. There is therefore a need for a more accurate assessment of the pain nature for each patient.

Other solutions analyse the underlying mechanisms of pain such as quantitative sensory testing, laser-evoked potentials, microneurography, functional brain imaging, skin biopsy or current perception thresholds. However, these solutions are complex, time-consuming, require an expert to be applied or are still not mature enough to be applied in standard clinical practice.

It is of relevance the psychological factors in the assessment of patients with FBSS who are candidates for SCS. High rates of undiagnosed psychological distress have been shown in patients referred for SCS, and a positive relationship exists between psychological factors and poor treatment outcome. Particularly, presurgical somatization, depression, anxiety, and poor coping are the most indicative psychological factors in predicting poor response to lumbar surgery and SCS.

There is therefore a need for a simple and relatively fast solution for the assessment of patients with FBSS who are candidates for SCS, that can also relate to the psychological factors associated with these patients.

### SUMMARY OF THE INVENTION

A first aspect of the invention refers to a system for Current Perception Threshold (CPT) quantification of a user. The system is configured to generate data for classifying patients with failed back surgery syndrome (FBSS) eligible for spinal cord stimulation (SCS) treatment. The system comprises:
a) one or more electrode pairs;
b) a stimulator configured to provide a plurality of electrical stimuli between the pair of electrodes of each of the one or more electrode pairs, wherein the plurality of electrical stimuli comprises a plurality of AC signals of a frequency from a predefined set of frequencies;
c) a feedback element configured to determine whether the electrical stimuli is felt by the user; and
d) a processor, wherein the processor is configured to control the stimulator and receive an indication from the feedback element whether the electrical stimuli is felt by the user;

The one or more electrode pairs are provided on a substrate for placing the electrode pairs to the skin of the user and the system is further configured to trigger the electrical stimuli of the buttock, the lumbar, the thigh and/or the calf skin areas of the user through the one or more of electrode pairs, wherein each electrode pair defines a sub-area within the area. The processor is further configured to:
i. for each frequency of the set of frequencies for each of a plurality of electrode pair positions, to receive an indication of the location of the electrode pair positions on the buttock, the lumbar, the thigh and/or the calf, and control the stimulator to provide two or more electrical stimuli at the frequency and electrode pair positions, the two or more electrical stimuli having different electrical intensities;
ii. for each electrical stimulus, receive an indication by the feedback element whether the electrical stimuli were felt by the user;
iii. for each frequency at each electrode pair position, determine a threshold value for the lowest electrical intensity felt by the user based on the received indications for each electrical stimulus; and
iv. for each frequency for the buttock, the lumbar, the thigh and/or the calf, calculate an average threshold value indicative of the lowest electrical intensity felt by the user, based at least in part on the threshold values determined in iii).

A second aspect of the invention refers to a computer-implemented method to generate data for classifying patients with failed back surgery syndrome (FBSS) eligible for spinal cord stimulation (SCS) treatment. The method comprises the steps:
a) receiving data obtained from one or more electrode pairs placed at a plurality of electrode pair positions on a user, wherein for each frequency of a plurality of frequencies at each electrode pair position, two or more electrical stimuli at the frequency and having different electrical intensities were provided, wherein the data comprises, for each electrical stimuli and electrode pair position: the location of the electrode pair position on the buttock, the lumbar, the thigh and/or the calf; the frequency of the electrical stimulus, the electrical intensity of the electrical stimulus and an indication of whether the electrical stimulus was felt by the user;
b) for each frequency for the buttock, the lumbar, the thigh and/or the calf, determining a threshold value for the lowest electrical intensity sensitive to the user based on the received indication for each electrical stimulus; and
c) for each frequency for the buttock, the lumbar, the thigh and/or the calf, calculating an average threshold value indicative of the lowest electrical intensity felt by the user, based at least in part the threshold values determined in b).

A third aspect of the invention refers to a computer program product comprising instructions which, when the program is executed by a processor, cause the computer to carry out the steps of the method according to any embodiment of the second aspect of the invention.

### BRIEF DESCRIPTION OF THE DRAWINGS

Embodiments will now be explained in detail, by way of non-limiting example only, with reference to the accompanying figures described below.
Figure 1: shows a schematic diagram of a system for Current Perception Threshold quantification according to one or more embodiments.
Figure 2: shows a schematic diagram showing different types of electrode pair configurations according to one or more embodiments.
Figure 3: shows a schematic diagram of a method for generating data for classifying patients with failed back surgery syndrome according to one or more embodiments.
Figure 4: shows a schematic diagram for determining a threshold value according to one or more embodiments.
Figure 5: shows a schematic diagram for determining a reference range of electrical current according to one or more embodiments.
Figure 6: shows a schematic diagram of a method for generating data for classifying patients with failed back surgery syndrome according to one or more embodiments.
Figure 7: shows a schematic diagram for a method of prognosticating the outcome of a patient with failed back surgery syndrome (FBSS) undergoing a spinal cord stimulation (SCS) according to one or more embodiments.
Figure 8 shows a schematic diagram of a computer program according to one or more embodiments.
Figure 9 shows the mean and standard deviation comparison charts between the surgery and no-surgery groups for average sensitivity thresholds for different locations tested and at three different frequencies, using a system for Current Perception Threshold quantification according to one or more embodiments.
Figure 10 shows the mean and standard deviation comparison charts between the surgery and no-surgery groups for the standardized average sensitivity thresholds for different locations tested and at three different frequencies, using a system for Current Perception Threshold quantification according to one or more embodiments.
Figure 11 shows the mean and standard deviation comparison charts between "excellent" improvement-labelled subjects and the rest of the subjects for standardized average sensitivity thresholds for different locations tested and at three different frequencies using a system for Current Perception Threshold quantification according to one or more embodiments.
Figure 12 shows the mean and standard deviation comparison charts between "excellent" and "very good" improvement labelled subjects and the rest of the subjects for standardized average sensitivity thresholds for different locations tested and at three different frequencies, using a system for Current Perception Threshold quantification according to one or more embodiments.
Figure 13A shows an exemplary graph of a calculated threshold value against stimuli frequency for a plurality of electrode pair positions.
Figure 13B shows an exemplary map of calculated threshold values for a given frequency for a plurality of electrode pair positions.

### DETAILED DESCRPTION

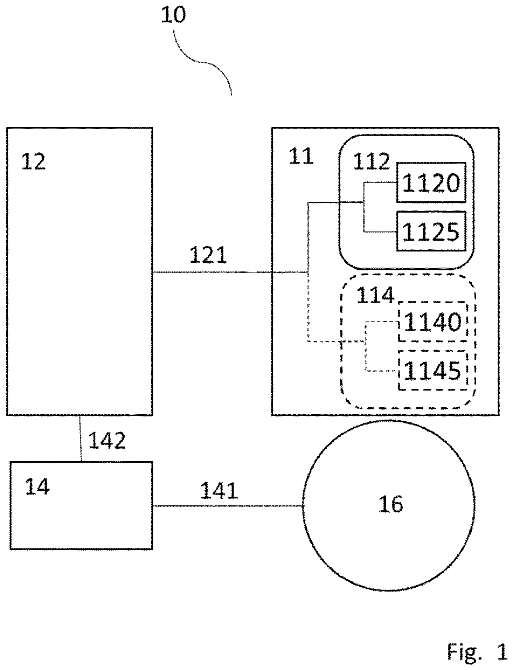

Figure 1 shows a system 10 for Current Perception Threshold (CPT) quantification of a user, the system configured to generate data for classifying patients with failed back surgery syndrome (FBSS) eligible for spinal cord stimulation (SCS) treatment according to one or more embodiments. The system comprises one or more electrode pairs 112, 114; a stimulator 12 configured to provide a plurality of electrical stimuli between the pair of electrodes 1120, 1125 and 1140, 1145 of each of the one or more electrode pairs 112, 114 respectively, wherein the plurality of electrical stimuli comprises a plurality of AC signals of a frequency from a predefined set of frequencies; a feedback element 16 configured to determine whether the electrical stimuli is felt by the user; and a processor 14, wherein the processor 14 is configured to control the stimulator 12 and receive an indication from the feedback element 16 whether the electrical stimuli is felt by the user.

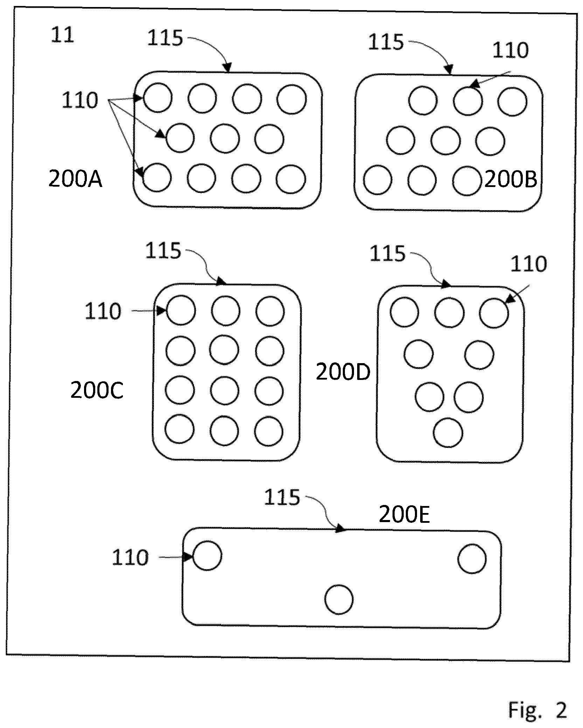

It is noted that in the system 10 the electrode pair 114 is optional. Each electrode pair 112, 114 is composed of two electrodes 1120, 1125 and 1140, 1145 respectively, that allow an electrical current to flow from one to another. It is noted that the direction of this flow can be in either direction. In some embodiments the electrodes may be equally shaped while in other embodiments these electrodes may take different forms. For example, one electrode maybe be circular while the other may be ring-shaped, and the electrodes may be concentric. In some embodiments the electrodes may be placed one next to the other; while in other embodiments, the electrodes may be placed in different positions relative one to the other. For example, for the circular and ring-shaped electrodes, the circular electrode may be placed inside the ring-shaped electrode, so that the current flow goes from the inner circular electrode to the outer ring-shaped electrode or from the outer ring-shaped electrode towards the inner circular electrode. It is noted that the system 10 may comprise different electrode pair types. The possible disposition of the electrode pairs when the system comprises a plurality of electrode pairs will be further disclosed with reference to Figure 2.

The stimulator 12 is configured to provide a plurality of electrical stimuli to the electrodes through electrical means 121 such as wires. The electrical means 121 may comprise more than one wire comprising a plurality of conducting paths, wherein respective conducting paths are connected to respective electrodes of each electrode pair 112, 114. The stimulator 12 may be able to select one electrode pair 112, 114 from a plurality of electrode pairs. To do so, the stimulator 12 may comprise an electrical switch (not shown). The electrical switch may be internal or external to the stimulator 12 and the anode and cathode wires of each electrode pair 112, 114 may be common between the stimulator 12 and the electrical switch. The electrical means 121 may further comprise a control wire that allows the stimulator 12 to send control signals to the electrical switch to select the electrode pair the stimulus should be provided to. The stimulator 12 may comprise any suitable electrical controller for controlling which electrode pair an electrical stimulus is sent to. The stimulator 12 may comprise any suitable power source that powers it in AC or DC and the stimulator 12 may comprise a DC-AC inverter to convert DC current into the stimuli AC current. The stimulator 12 may comprise a pulse generator so that the stimuli can take different desired waveforms such as sinusoidal or squared waves. In some embodiments, the stimulator 12 may be connectable to and powered by an external power source. The AC signals current may be therefore provided by an internal or an external power source. The plurality of electrical stimuli comprises a plurality of AC signals of a frequency from a predefined set of frequencies. These frequencies are determined taking into consideration the nerve fibres of interest to stimuli. For example, the plurality of frequencies may comprise one or more of: a suitable frequency for preferentially stimulating the C-type fibres (for example 5Hz), a suitable frequency for preferentially stimulating the Aδ-type fibres (for example 250Hz) and a suitable frequency for preferentially activating the Aβ-type fibres (for example 2000Hz). It is noted that the plurality of frequencies may comprise other frequencies to stimulate other or the same nerve fibres in the same or different proportions. The intensity of the stimuli may be varied by varying the voltage amplitude or the current amplitude of the electrical stimuli (i.e. the stimulator 12 may be a variable current or voltage source). When the current amplitude is varied, the current amplitude may be comprised between greater than 0 and 10 mA. The stimulator 12 as a current source may have any suitable maximum output voltage, such as 180V.

The term "preferentially" refers to any frequency which predominantly cause the referred fibre-type to be stimulated over other nerve-types (i.e. the majority of the electrical stimuli is converted into electric stimulation of the preferred fibre-type over the other fibre-types), even if the frequency also causes other fibre types to be stimulated. In particular, the objective of the apparatus is to obtain sensory thresholds for particular fibre-types. It is noted that the stimulation of one fibre-type or another does not depend exclusively on the frequency of the stimulus but on the duration of and electrical intensity of the stimulus, as well as the type of current and the waveform and the electrode shape and size. Given a particular setup of the apparatus (waveform, electrode size and shape etc.) it can be determined in a number of different manners which frequencies cause preferential stimulation of which fibre-types, for example by a preliminary observation in a group of patients for a number of different frequencies to determine suitable frequencies for the preferential stimulations.

In some embodiments, the predetermined set of frequencies comprises: a first frequency for preferentially stimulating C-type fibres, the first frequency preferably comprised between 2 and 6 Hz; and/or a second frequency for preferentially stimulating Aδ-type fibres, the second frequency preferably comprised between 200 and 300 Hz; and/or a third frequency for preferentially stimulating Aβ-type fibres, the third frequency preferably comprised between 1500 and 3000 Hz. It is noted that other frequencies may be used to stimulate each of the fibre nerves types depending on the particular setup used.

The feedback element 16 is configured to determine whether the electrical stimuli is felt by the user. The feedback element 16 may be any suitable feedback element suitable for detecting whether the electrical stimuli is felt by the user. There are many feedback elements that can be configured in such way, that can comprise both active feedback elements, in which the user actively indicates whether the stimulus is felt, and/or passive feedback elements in which the element detects whether the stimuli were felt by the user without the user actively or consciously indicating so. Active elements may comprise any interface the user may interact with such as: a touch screen or other display device with guided user interface (GUI); or a button or switch connected to a sensor configured to detect when the button or switch is pressed; a pressure sensor; a lever; a microphone and associated noise detection module (implemented in hardware and/or software) configured to detect a noise indicating that the stimulus is felt, optionally comprising a voice recognition software; a camera and associated image processing module (implemented in hardware and/or software) configured to detect any suitable visual indication from the user that the stimuli was felt; or any other suitable means . Passive elements may comprise any element capable of determining whether the electrical stimuli are felt by the user without any active input by the user. They may comprise, for example: a pupil tracking device with an associated pupil tracking module (implemented in hardware and/or software) configured to detect a pupil dilation or contraction associated with the electrical stimulus being felt; a facial recognition device with an associated face tracking module (implemented in hardware and/or software) configured to detect a facial pose associated with the electrical stimulus being felt; as well as any other means that may be able to do so. The feedback element 16 is further connected to the processor 14 through the connection means 141, that may be either physical means such as a wire or a wireless connection such as a Bluetooth or Wi-Fi connection.

The processor 14 may comprise one or more processing units, such as a microprocessor, GPU, CPU, multi-core processor or similar, and may be internal or external to the stimulator 12 or distributed between processors of different devices. The processor 14 is configured to control the stimulator 12 by indicating the activation of the stimulation and optionally controlling one or more characteristics of the stimuli such as the intensity, frequency, location, waveform or duration. The processor 14 is therefore connected to the stimulator 12 through the connection means 142 that may be either physical means such as a wire or a digital electromagnetic connection such as a Bluetooth or Wi-Fi protocols. The processor 14 is also configured to receive an indication from the feedback element 16 whether the electrical stimuli is felt by the user through the connection means 141. Optionally, in some embodiments the system comprises a memory associated with the processor 14 configured to save all the information the processor 14 processes and obtain the saved information. The memory may comprise one or more volatile or non-volatile memory devices, such as DRAM, SRAM, flash memory, read-only memory, ferroelectric RAM, hard disk drives, floppy disks, magnetic tape, optical discs, or similar. Alternatively, the stimulator 12 may comprise a communication interface (not shown) configured to communicate with one or more external devices, and data obtained by the stimulator 12 may be sent to the one or more external devices, such as an external computer, server, mobile device or distributed computing system such as the cloud. It is noted that in some embodiments the processor 14 and optionally the memory, are comprised within the stimulator 12.

The system 10 is further configured to trigger the electrical stimuli of the buttock, the lumbar, the thigh and/or the calf skin areas of the user through the one or more of electrode pairs, wherein each electrode pair defines a sub-area within the area. Therefore, the stimulator 12 provides stimuli to the body parts affected by the FBSS. Furthermore, the system 10 may be configured to trigger the electrical stimuli of a non-FBSS affected area, such as an upper limb, in order to calculate reference threshold values for the electrical stimuli as disclosed in further detail herein.

The processor 14 is further configured to receive an indication of the location of the one or more electrode pairs 112, 114 on the buttock, the lumbar, the thigh and/or the calf, for a plurality of electrode pair positions (i.e. whether the one or more electrode pairs are placed on the buttock, lumbar thigh or calf, and optionally the sub-area within the area of the buttock, lumbar, thigh or calf on which each electrode pair is placed). This indication may be received from a user interface of an external device communicatively connected to the processor 14 (for example via an app of a mobile device or from a guided user interface GUI of an external device or a web portal), or may be provided via a user interface of a device comprising the processor 14.

The processor 14 is further configured to, for each electrical stimulus, receive an indication by the feedback element whether the electrical stimuli was felt by the user, and for each frequency at each electrode pair position, determine a threshold value for the lowest electrical intensity felt by the user based on the received indications for each electrical stimulus, as described in further detail herein.

The processor 14 is further configured to, for each frequency for the buttock, the lumbar, the thigh and/or the calf, calculate an average threshold value indicative of the lowest electrical intensity felt by the user, based at least in part on the threshold values determined, as described further herein.

For example, when the system 10 comprises a plurality of electrode pairs, a practitioner may place the plurality of electrode pairs on the buttock, lumbar, thigh or calf in a predetermined position. Once the practitioner determines that the plurality of electrode pairs are correctly positioned, the practitioner provides the indication about where the plurality of electrode pairs are placed (e.g. by selecting the appropriate option on the user interface of "buttock", "lumbar", "thigh" or "calf", or by selecting an area on a schematic diagram of a patient body corresponding to the electrode pair position). The indication may include specific indications regarding the position of each of the plurality of electrode pairs within the area of the buttock, lumbar, thigh or calf. These specific indications may be provided manually by the practitioner or may be determined by the processor 14 according to pre-programmed instructions (i.e. in embodiments where the plurality of electrode pairs have a fixed spatial relation to one another, such as on a single substrate, such that, when the practitioner indicates that the substrate is correctly placed on the buttock, lumbar, thigh or calf, the processor 14 calculates the position of each electrode pair according to the pre-programmed instructions related to the fixed position of the electrode pairs on the substrate). Once the indication is received, the system 10 may provide the electrical stimuli and determine if they were felt by the user as described in further detail herein. The practitioner may then place the plurality of electrode pairs on another of the buttock, lumbar, thigh or calf and again provide an indication about where the electrode pairs are placed as before. The system 10 may again provide the electrical stimuli and determine if they were felt by the user. This may be repeated for two, three or all of the buttock, lumbar, thigh or calf or may only be performed for one of the buttock, lumbar, thigh or calf. The same process may be performed for one or more electrode pair positions placed on a non-FBSS-affected area, such as an upper limb, to generate a set of reference threshold values for a non-FBSS-affected area.

In embodiments where the system 10 comprises only one electrode pair, a practitioner may place the electrode pair on the buttock, lumbar, thigh or calf in a predetermined position. Once the practitioner determines that the electrode pair is correctly positioned, the practitioner provides the indication about where the electrode pair is placed (e.g. by selecting the appropriate option on the user interface of "buttock", "lumbar", "thigh" or "calf", or by selecting an area on a schematic diagram of a patient body corresponding to the electrode pair position). The indication may also include a specific indication regarding the position of the electrode pair within the area of the buttock, lumbar, thigh or calf. This specific indication may be provided manually by the practitioner or may be determined by the processor 14 according to pre-programmed instructions (i.e. the practitioner places the electrode pair in a specific position within the area of the buttock, lumbar, thigh or calf and confirms that it is placed in the area corresponding to the area saved in the pre-programmed instructions). Once the indication is received, the system 10 may provide the electrical stimuli and determine if they were felt by the user as described in further detail herein. The practitioner may then place the electrode pair on another area within the buttock, lumbar, thigh or calf, and again provide an indication about where the electrode pair is placed as before. The system 10 may again provide the electrical stimuli and determine if they were felt by the user. The practitioner may place the electrode pair on another of the buttock, lumbar, thigh or calf and again provide an indication about where the electrode pairs are placed as before. The system 10 may again provide the electrical stimuli and determine if they were felt by the user. This may be repeated for two, three or all of the buttock, lumbar, thigh or calf or may only be performed for one of the buttock, lumbar, thigh or calf. The same process may be performed for one or more electrode pair positions placed on a non-FBSS-affected area, such as an upper limb, to generate a set of reference threshold values for a non-FBSS-affected area.

Accordingly, whether the system 10 comprises one electrode pair or a plurality of electrode pairs, the practitioner is able to place the electrode pairs at plurality of electrode positions within each of one or more of the buttock, lumbar, thigh and calf. The plurality of electrode pair positions may be provided simultaneously in the case where the system 10 comprises a plurality of electrode pairs (i.e. each electrode pair corresponds to an electrode pair position), or a single electrode pair may be place consecutively in a plurality of electrode pair positions (i.e. the single electrode pair is placed in a plurality of electrode positions). In either case, the system 10 is configured provide multiple electrical stimuli at each electrode pair position and determined if each of the electrical stimuli were felt by the user, according to the methods disclosed herein.

In embodiments where the system 10 is further configured for classifying patients with failed back surgery syndrome (FBSS) by mental condition and wherein the system 10 comprises one electrode pair or a plurality of electrode pairs, the practitioner may place the one pair or plurality of electrode pairs on a non-FBSS-affected area in one or more predetermined electrode pair positions on the non-FBSS-affected area. The plurality of electrode pair positions may be provided simultaneously in the case where the system 10 comprises a plurality of electrode pairs (i.e. each electrode pair corresponds to an electrode pair position), or a single electrode pair may be place consecutively in a plurality of electrode pair positions (i.e. the single electrode pair is places in a plurality of electrode positions). Once the practitioner determines that the one pair or plurality of electrode pairs are correctly positioned, the practitioner provides the indication about where the plurality of electrode pairs are placed, i.e. the practitioner may indicate that the plurality of electrode pairs are positioned in the predetermined position of the non-FBSS-affected area. Then the system 10 is configured to provide multiple electrical stimuli at each electrode pair position and determined if each of the electrical stimuli were felt by the user, according to the methods disclosed herein. The determination of the thresholds in a non-FBSS-affected area may be then used a reference as described in further detail herein.

It is noted, that as shown in the Example, the data obtained through the system 10 can be correlated to the mental condition of the user and therefore does not only provide a CPT quantification, but it also provides data that can provide useful information regarding the mental condition of the user.

Figure 2 shows a plurality of sets of electrode pairs of system 10 according to one or more embodiments. The one or more electrode pairs 110 are provided on a substrate 115 for placing the electrode pairs to the skin of the user. The substrate 115 of the different electrode pair sets configure the electrodes to be spatially located in different locations relative to each other, allowing for a desired distribution according to the area of the body the set of electrode pairs are configured to be placed. For example, the substrate 115 may distribute the electrode pairs 110 in such way that they cover most of the buttock, the lumbar, the thigh and/or the calf skin areas of the user with the electrode pairs distributed about the area of the body. In some embodiments, the substrate 115 may distribute the electrode pairs 110 by considering the nerve distribution of a non-FBSS-affected skin area, such as the upper limb. It is noted that each electrode pair 110 comprises two electrodes and that each electrode may take different shapes and sizes and dispositions relative to each other as previously mentioned. In some embodiments, the substrate may help fixing the electrodes in the desired place and may include an adhesive surface or any affixing element. In some embodiments the substrate is configured to be detachable from the electrode pairs, so that in use it can be used as a template or stencil for the electrodes but not applied or fixed to the user. The set of electrode pairs 200A comprises an array of three rows of evenly distributed electrode pairs, preferably in a 4-3-4 formation. The set 200A is particularly adapted to stimulate the fibres in variety of sub-areas of the lumbar. The set of electrode pairs 200B comprises an array of three rows of evenly distributed electrode pairs, preferably in a 3-3-3 formation forming a rhombus shape. The set 200B is particularly adapted to stimulate the fibres in variety of sub-areas of the buttock. The set of electrode pairs 200C comprises an array of four rows of evenly distributed electrode pairs, preferably in a 3-3-3-3 formation forming a rectangular shape. The set 200C is particularly adapted to stimulate the fibres in variety of sub-areas of the thigh. The set of electrode pairs 200D comprises an array of four rows of electrode pairs, preferably in a 3-2-2-1 formation. The set 200D is particularly adapted to stimulate the fibres in variety of sub-areas of the calf. The set of electrode pairs 200E comprises an array of three electrode pairs. The set 200E is particularly adapted to stimulate the fibres on the upper arm (e.g. in the biceps or triceps area), the lower arm (e.g. the ulna or radii area) and the hand. The system 10 may comprises a plurality of sets of electrode pairs having different formations adapted to stimulate a plurality of the lumbar, buttock, thigh, calf and non-FBSS-affected area such as an upper limb. The plurality of sets of electrode pairs may be removably connectable to the stimulator 12 by electrical connectors, so that the practitioner is able to connect the appropriate set of electrodes for stimulating a particular area of the body.

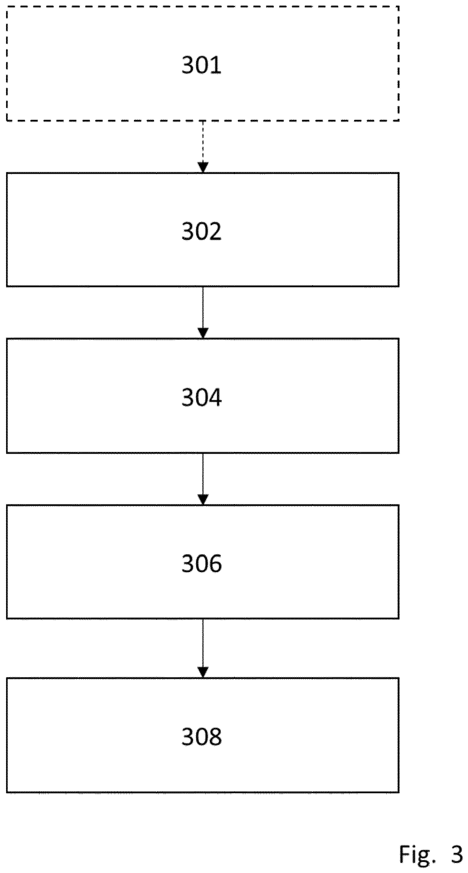

Figure 3 shows a diagram of several processing steps the processor 14 of the system 10 is configured to perform, according to one or more embodiments. The processor 14 is configured to take the step 302 wherein, for each frequency of a set of predefined frequencies for each of a plurality of electrode pair positions, an indication of the location of the electrode pair positions on the buttock, the lumbar, the thigh and/or the calf is received, the stimulator 12 is controlled to provide two or more electrical stimuli at each frequency for each electrode pair position, the two or more electrical stimuli having different electrical intensities (e.g. voltage or current amplitude intensities). It is noted that the stimulator 12 may be configured to provide a plurality of stimuli having the same intensity and frequency (for example a plurality of stimuli at a frequency F1 and intensity 11, and a plurality of stimuli at frequency F1 and intensity 12). The indication of the location of the electrode pair positions may be provided through electronic means from a user interface located in a separate device. The user interface may comprise buttons, knobs, sliders, levers, wheels, a touchscreen, and so on, that the user can use to indicate the location of the electrode pair positions. In some embodiments, a plurality of predetermined electrode pair positions may be saved in a memory of the system 10 and the indication may be a selection of the relevant predetermined electrode pair positions which corresponds to the electrode pair position on the patient. The indication may simply be a confirmation indication made via a user interface that the electrode pair position is a predetermined electrode pair position saved in the memory. In some embodiments, the system 10 comprises the user interface either as a separate element or comprises within any of the other elements of the system. The stimulator 12 is controlled through the means 142 and the processor 14 may indicate the location of the electrode pair position to stimulate and the frequency, time and/or intensity of the stimuli to be applied.

The processor 14 is also configured to take the step 304, wherein for each electrical stimulus, an indication is received by the feedback element 16 whether the electrical stimuli was felt by the user. The processor 14 may receive the indication through the means 141. It is noted that in some embodiments the feedback element 16 may provide feedback only when the electrical stimuli was felt by the user, while in other embodiments the feedback element 16 may provide a positive feedback when the electrical stimuli was felt by the user, and a negative feedback when the electrical stimuli was not felt by the user, or feedback element 16 may provide feedback only when the electrical stimuli was not felt by the user.

The processor 14 is further configured to take the step 306, wherein for each frequency at each electrode pair position, a threshold value is determined for the lowest electrical intensity felt by the user based on the received indications for each electrical stimulus. This threshold value may be determined by configuring the processor 14 to process the information regarding the stimuli provided, including its frequency and intensity, and the indication whether the electrical stimuli was felt by the user. In some embodiments the processor 14 is further configured to determine, for each frequency at each electrode pair position the threshold value of step 306 is determined by selecting the lowest electrical intensity indicated as being felt by the user.

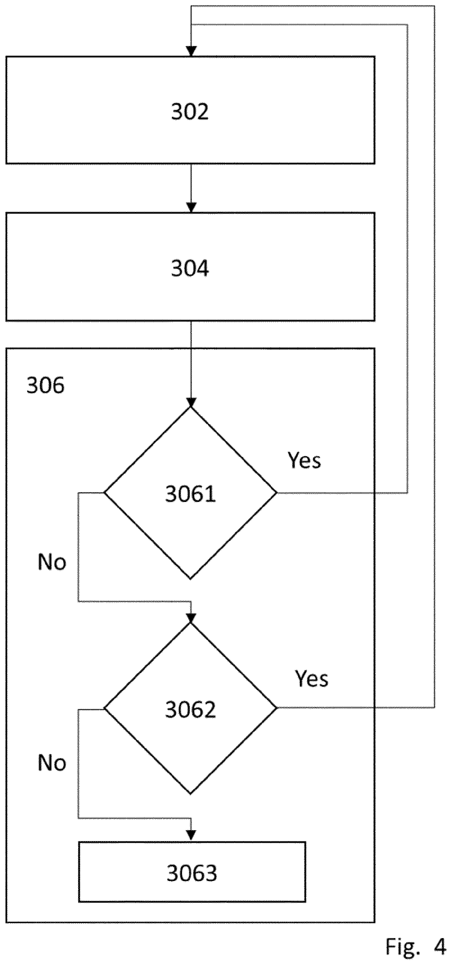

In other embodiments, the threshold value of step 306 is determined by further analysing the lowest electrical intensity indicated as being felt by the user. In some embodiments, the threshold value of step 306 is determined by selecting the lowest electrical intensity indicated as being felt by the user wherein the immediately higher electrical intensity is also indicated as being felt by the user. In some embodiments, wherein a plurality of stimuli has been provided for each frequency at each electrode pair position at each current intensity, the threshold value of step 306 is determined by selecting the lowest electrical intensity indicated as being felt by the user wherein the threshold value for the lowest electrical intensity is indicated as felt by the user at least twice; or by selecting the lowest electrical intensity indicated as being felt by the user wherein the threshold value for the lowest electrical intensity is indicated as felt by the user more than half of the times; or by selecting the lowest electrical intensity indicated as being felt by the user wherein the threshold value for the lowest electrical intensity is indicated as felt by the user all the times. A further embodiment of step 306 will be detailed with reference to Figure 4.

The term "immediately higher" when referring to an electrical intensity is understood as the stimuli provided to the same electrode pair position and at the same frequency with the next superior intensity value from the plurality of intensities provided, without any other stimuli having an intensity value between them.

The processor 14 is further configured to perform step 308, wherein for each frequency for the buttock, the lumbar, the thigh and/or the calf, an average threshold value is calculated for the lowest electrical intensity felt by the user, from at least the threshold values determined in the step 306. The calculated average threshold values may be non-standardised, i.e. they are not standardised in relation to threshold values obtained in a non-FBSS-affected area, or they may be standardised averages obtained in relation to threshold values obtained in a non-FBSS-affected area. In some embodiments, both non-standardised and standardised average threshold values are obtained. In some embodiments, the average threshold value may be computed by obtaining the arithmetic mean of the threshold values from the electrode pair positions for the buttock, the lumbar, the thigh and/or the calf.

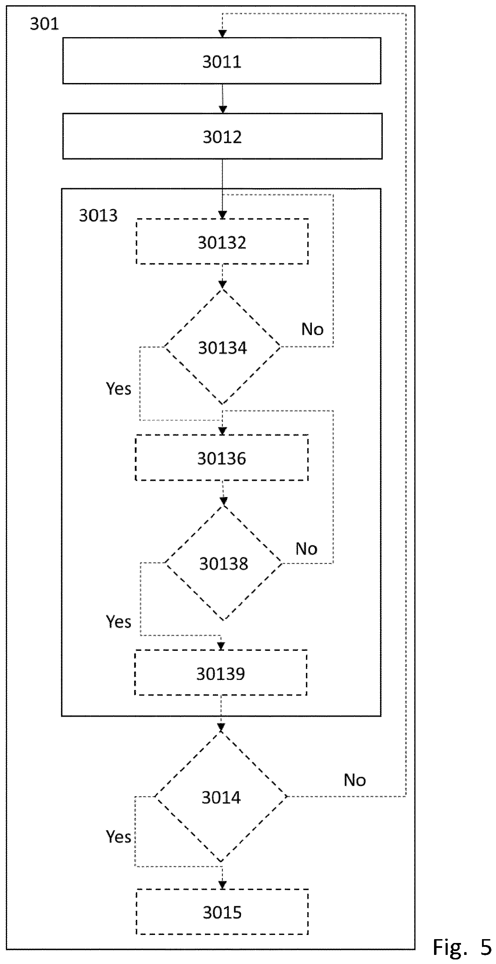

In some embodiments, the processor 14 is configured to take the step 301 of determining a reference range of electrical intensity. This step will be further described with reference to Figure 5.

In some embodiments, the processor 14 is further configured to obtain a standardised average threshold value with respect to a non-FBSS-affected electrode position; wherein the standardised average threshold value is indicative of the difference between a threshold value and the non-FBSS threshold value. The processor 14 is therefore configured to take the steps of: a) for each frequency of the set frequencies for a non-FBSS-affected electrode pair position to activate the stimulator 14 providing two or more electrical stimuli at the frequency and non-FBSS-affected electrode pair position, the two or more electrical stimuli having different electrical intensities; b) for each electrical stimulus at the non-FBSS-affected electrode pair position, determining by the feedback element 16 whether the electrical stimuli was felt by the user; c) for each frequency at the non-FBSS-affected electrode pair position, determining a non-FBSS threshold value for the lowest electrical intensity felt by the user based on the received indications for each electrical stimulus; and d) calculate a standardised threshold value indicative of the difference between an average threshold value in an FBSS-affected area and the non-FBSS threshold value for one or more frequencies at one or more electrode pair positions. The standardised average threshold value may for example be a subtraction of the non-FBSS threshold value from the FBSS threshold value, or a ratio may be calculated. It is noted that in the embodiments wherein electrode pairs are configured to be placed on a non-FBSS-affected area in a predetermined position all the previous consideration regarding the activation of the stimulator 12 (step 302), the determination whether the stimuli was felt by the user by the feedback (step 304) the threshold value determination (step 306) and the average threshold value calculation (step 308) are equivalently applicable to these steps the processor 14 is configured to take.

The terms "non-FBSS-affected electrode pair position" and "non-FBSS-affected area" are referred to a position/area over the skin surface over a place where FBSS related pain is not located. This may comprise, for example, the upper limb or the shoulder.

In the Example, it has been shown that this standardised average threshold may be reliable source of information to classify patient with failed back surgery syndrome (FBSS) eligible for spinal cord stimulation (SCS) treatment taking into account the mental condition of the patient.

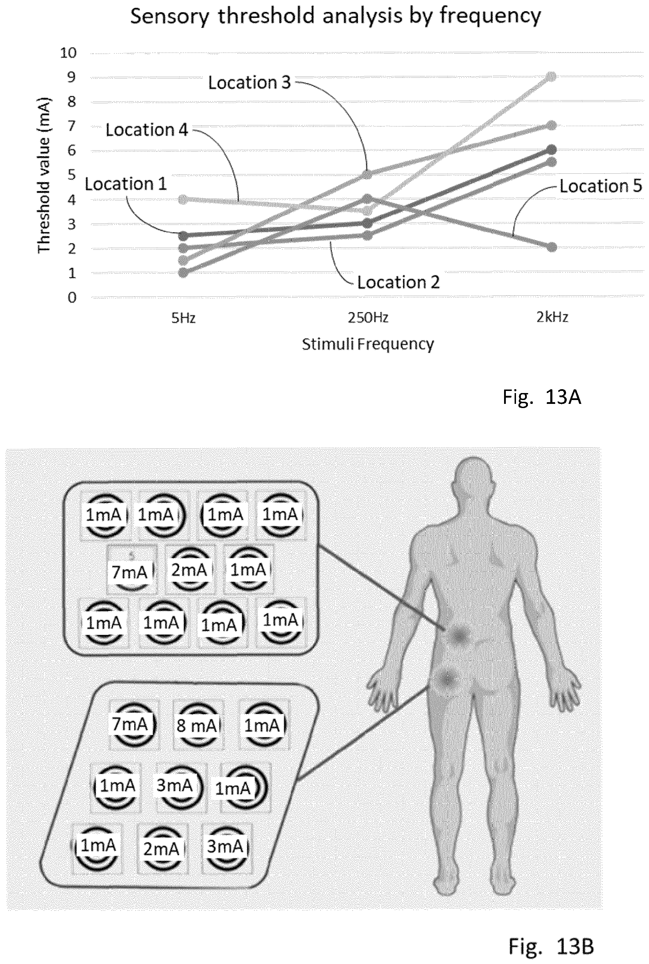

In some embodiments, wherein the processor 14 is further configured to generate a set of data from the determined CPT wherein the data indicates one or more of the following: the threshold value as a function of stimuli frequency for the plurality of electrode pair positions; and one or more maps of the threshold value as a function of electrode pair position, each map indicating the threshold value for a single frequency. Therefore, the threshold values may be grouped by frequencies, or by location and may be filtered to provide information reading only a location, frequency or intensity of interest. This may help the practitioner to analyse the data in different way and determine the classification of the patient. Optionally, in some embodiments, the system 10 further comprises a display device configured to display the set of data, wherein the processor 14 is further configured to display the set of data on the display device, as shown in Figures 13A and 13B. Fig. 13A shows the calculated threshold values in mA for each electrode pair position (locations 1-4), wherein the threshold for each electrode pair position is indicated by a unique colour with markers at the threshold value for each frequency and connecting lines between the markers. Fig. 13B shows the calculated threshold values in mA for particular frequencies, illustrated on a graphic of the electrode pair positions.

Figure 4 shows a diagram showing further steps the processor 14 is configured to take in order to improve the system's 10 reliability when abnormal inputs are received, according to one or more embodiments. For step 306, the processor 14 may be configured to perform the step 3061 of checking if, for all the intensities provided to the user in a particular electrode pair position and frequency, the indication received by the feedback element 16 whether all the electrical stimuli were felt by the user is positive, i.e. indicates the user has felt it all of the stimuli, the processor 14 is configured to repeat the steps 302 and 304 for that electrode pair location and frequency with a lower set of intensities. Moreover, if that is not the case the processor 14 is further configured to perform the step 3062 of checking if for all the intensities provided to the user the indication received by the feedback element 16 whether none of the electrical stimuli were felt by the user is negative, i.e. indicates the user has not felt any of the stimuli, the processor 14 is configured to repeat the steps 302 and 304 for that electrode pair location and frequency with a higher set of intensities. If that is not the case, the processor 14 is then configured to take the step 3063, wherein it determines a threshold value for the lowest electrical intensity felt by the user based on the received indications for each electrical stimulus as previously described in step 306. It is noted that steps 3061 and 3062 may be interchangeable and therefore the processor 14 may be configured to first check if none of the stimuli were felt by the user and then check if all the stimuli were felt by the user, through the feedback element 16. Steps 3061 and 3062 may instead be comprised in a single step checking whether both positive and negative indications were received, and if so proceeding to step 3063, if not repeating steps 302 and 304 with a higher and/or lower set of intensities. It is also noted that in some embodiments, the processor 14 may not be configured to take the steps 3061 or 3062.

Figure 5 shows a diagram of a previous step 301 the processor 14 is further configured to make in order to determine a reference range of electrical intensity according to one or more embodiments. The processor 14 may be configured to take the step 3011 of receiving an indication of the location of an electrode pair position and controlling the stimulator 12 to provide one or more preliminary stimuli to the user at the electrode pair position. In some embodiments, this may be done for one or more frequencies from a predetermined set of frequencies and for a reference electrode pair position on the buttock, the lumbar, the thigh and/or the calf. It is noted that in some embodiments the reference electrode pair is predetermined while in other embodiments the reference electrode pair is randomly chosen from the electrode pairs available. The processor 14 may be then configured to take the step 3012 of receiving one or more indications via the feedback element 16 whether the electrical stimuli were felt by the user. The processor may be then configured to take step 3013, of determining a reference range of electrical intensity based in the received indications. In some embodiments, this is done for each frequency at the reference electrode pair position whilst in others the same reference range is used for all frequencies. It is noted that for the embodiments wherein electrode pairs have been also placed on a non-FBSS-affected area in a predetermined position, the processor may be configured to take the steps as described above.

In Figure 5 an optional set of steps of the configuration of the processor 14 to take step 3013 according to one or more embodiments are further represented. The reference range of electrical intensity may be then determined according to one or more embodiments as follows. The processor 14 may be configured to take step 30132, wherein a first plurality of stimuli sequentially ascending in electrical intensity is provided. The processor 14 may be then configured to take step 30136, wherein a second plurality of stimuli sequentially descending in electrical intensity is provided. The processor 14 may be then configured to take the step 30139, wherein the maximum and minimum for the reference range are selected to be the highest and lowest electrical intensities wherein a change in the received indications is noted. In some embodiments, the processor 14 is further configured to take step 30134, wherein if none of the indication via the feedback element 16 whether the electrical stimuli were detected, the step 30132 is repeated with higher intensities. In some embodiments, the processor 14 is further configured to take step 30138, wherein if all of the indication via the feedback element 16 whether the electrical stimuli were detected, the step 30136 is repeated with lower intensities. In some embodiments, the processor 14 is further configured to take step 3014, wherein if the reference range of electrical intensity is higher than a predetermined maximum range the steps 3011, 3012 and 3013 are repeated until the reference range is less than the predetermined maximum range, unless a maximum number of iterations is reached. For example, the processor 14 may be configured to repeat steps 3014 if the reference range of electrical intensity is higher than 0.5mA until the reference range is less than 0.5 mA, unless a maximum number of iterations, such as 3, is reached, wherein the processor is configures to take the step 3015 of considering such range as the reference range of electrical intensity.

In some embodiments, the system 10 further comprises a memory and the processor 14 is configured to store, for each of the electrical stimuli, data indicating one or more of the following: 1) the intensity, frequency, electrode pair position, the location of the electrode pair position on the buttock, the lumbar, the thigh and/or the calf of step 302; 2) whether the electrical stimulus has been felt by the user in step 304; 3) the threshold value of step 306; and 4) the average threshold value of 308. The memory is therefore configured to store the information provided by the processor 14 related to the steps 302, 304, 306 and 308, and to provide such information to the processor 14 when required by the processor 14. It is noted that for the embodiments wherein electrode pairs have been also placed on a non-FBSS-affected area in a predetermined position, the processor may be configured to store one or more of the following: the location of the electrode pair position of the non-FBSS-affected area; whether the electrical stimulus has been felt by the user; the threshold value; and the standardised average threshold value.

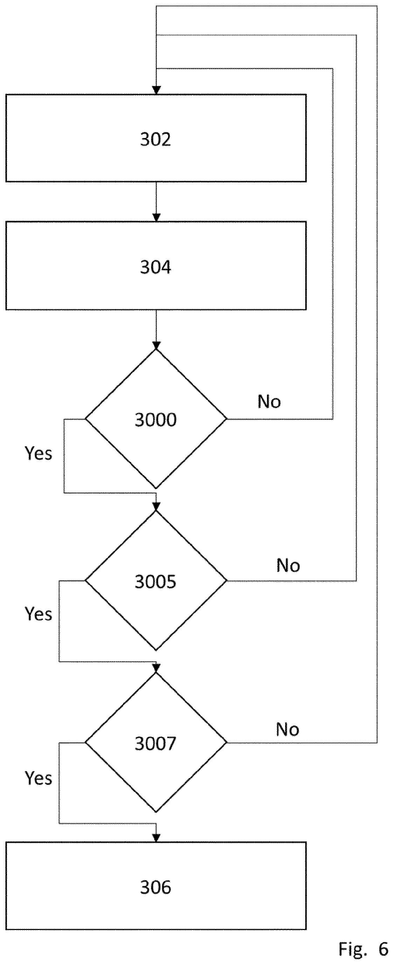

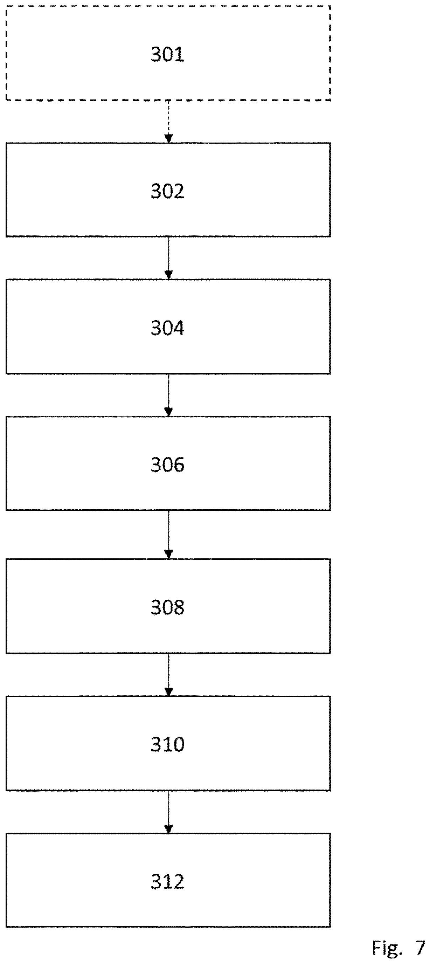

Figure 6 shows a diagram of a step 305 the processor 14 is further configured to, before taking the step 306, check if all the electrode pair positions of the plurality of pair positions and if all the frequencies of the plurality of frequencies the steps 302 and 304 have been applied, according to one or more embodiments. At step 3000, it is determined whether the processor 14 has performed the steps 302, 304 in all the electrode pairs positions. If not the processor 14 is configured to take the steps 302, 304 again with another electrode pair position. When at step 3000 it is determined that steps 302, 304 have been performed for all the electrode pairs positions, the processor proceeds to step 3005. In step 3005, the processor 14 determines whether steps 302, 304 have been performed for all the frequencies. If not the processor 14 is configured to take the steps 302, 304 again with another frequency. When at step 3005 it is determined that steps 302, 304 have been performed for all the frequencies, the processor proceeds to step 3007. In step 3007, the processor 14 determines whether steps 302, 304 have been performed for all predetermined electrical intensities. If not the processor 14 is configured to take the steps 302, 304 again with another frequency. When at step 3007 it is determined that steps 302, 304 have been performed for all the intensities, the processor proceeds to step 306. In some embodiments, the steps 3000, 3005, or 3007 may not be performed. It is noted that in some embodiments the processor 14 may be configured to take steps 3000, 3005, and/or 3007 in a specific order, for example first step 3000, then 3005 and then 3007 while in other embodiments the order may be first step 3007 then 3000 and then 3005 or maybe first step 3007 then 3005 and then 3000. It is also noted that the processor 14 may be configured to take steps 3000, 3005 and /or 3007 before steps 3004, 3008 or before any other step. Therefore, the processor 14 would be configured to take the previous steps again with the new electrode pair position and/or location. Figure 7 shows a diagram of the steps the processor 14 may be configured to take according to one or more embodiments, wherein the system 10 is configured to prognosticate the outcome of a patient with failed back surgery syndrome (FBSS) undergoing a spinal cord stimulation (SCS) treatment. Therefore, the system is configured to take the steps 302, 306, and 308 as previously described and optionally is configured to take the step 301 as previously described. The processor 14 is further configured to take step 3010, wherein the one or more average threshold values are compared with one or more predetermined standard average threshold values. Then the processor 14 is configured to take step 312, wherein the outcome of the patient undergoing an SCS treatment from the significant deviations found in the comparison is determined. As shown in the Example, the patients with an excellent response to SCS have significant higher averages CPTs compared to the rest, in the lumbar and the buttock regions at 5Hz, 250Hz and 2kHz.

It is noted that in step 3010, the processor 14 may be configured to compare either one or more average threshold values or one or more standardized average threshold values. The standardized average threshold values may be obtained from computing the difference between one or more average threshold value and a non-FBSS threshold value. As shown in the Example, the patients with a poor mental health outcome post-surgery according to the SF36-MCS questionnaire had a significantly greater standardized average threshold value in all the explored regions at 5 Hz. Therefore, the system 10 may be configured to prognosticate the mental health outcome of a patient with failed back surgery syndrome (FBSS) as a result of spinal cord stimulation (SCS) treatment.

A second aspect of the invention refers to a computer-implemented method according to one or more embodiments to generate data for classifying patients with failed back surgery syndrome (FBSS) eligible for spinal cord stimulation (SCS) treatment, wherein the method comprises the following steps:
a) receiving data obtained from one or more electrode pairs placed at a plurality of electrode pair positions on a user, wherein for each frequency of a plurality of frequencies at each electrode pair position, two or more electrical stimuli at the frequency and having different electrical intensities were provided, wherein the data comprises, for each electrical stimuli and electrode pair position: the location of the electrode pair position on the buttock, the lumbar, the thigh and/or the calf; the frequency of the electrical stimulus, the electrical intensity of the electrical stimulus and an indication of whether the electrical stimulus was felt by the user
b) for each frequency for the buttock, the lumbar, the thigh and/or the calf, determining a threshold value for the lowest electrical intensity sensitive to the user based on the received indication for each electrical stimulus; and
c) for each frequency for the buttock, the lumbar, the thigh and/or the calf, determining an average threshold value indicative of the lowest electrical intensity felt by the user, based at least in part on the threshold values determined in b).

It is noted that the received data from step a) could be obtained using a device according to any of the embodiments disclosed herein.

In some embodiments, the method further comprises the step of:
d) receiving data indicating a non-FBSS threshold value for the lowest electrical intensity felt by the user in a non-FBSS-affected area; and
e) generating data for classifying patients with FBSS eligible for SCS treatment from the determined CPT by calculating a standardised average threshold value indicative of the difference between the average of the threshold values of b) and the corresponding non-FBSS threshold value.

It is noted that the non-FBSS threshold value for the lowest electrical intensity felt by the user in a non-FBSS-affected area may be received through the steps a) to c) from non-FBSS-affected area, i.e. by
- receiving data obtained from one or more electrode pairs placed at a plurality of electrode pair positions on a non-FBSS-affected area of a user, wherein for each frequency of a plurality of frequencies at each electrode pair position, two or more electrical stimuli at the frequency and having different electrical intensities were provided, wherein the data comprises, for each electrical stimuli and electrode pair position: the location of the electrode pair position on the non-FBSS-affected area; the frequency of the electrical stimulus, the electrical intensity of the electrical stimulus and an indication of whether the electrical stimulus was felt by the user
- for each frequency, determining a threshold value for the lowest electrical intensity sensitive to the user based on the received indication for each electrical stimulus; and
- for each frequency, determining an average threshold value for the lowest electrical intensity felt by the user, from the threshold values determined.

The computer-implemented method may be performed on any suitable computing system comprising one or more processing units, such as a microprocessor, GPU, CPU, multi-core processor or similar, a server or a distributed computing system such as the cloud.

The term "standardise" in the present invention may be understood as adjusting different values by comparing them to a norm or a standard value, so that the standardised values are indicative of the amount of deviation from the norm. This comparison may comprise normalising using the standard value as the mean or calculating the difference to the standard value, either absolute or relative. In embodiments disclosed herein, the standardisation may be performed by subtracting the non-FBSS average threshold value from the average threshold values of the FBSS-affected areas (buttock, lumbar, thigh and/or calf), or a ratio of the calculated threshold values of the FBSS-affected areas to the non-FBSS average threshold value may be calculated.

In the Example, it has been shown that this standardised average threshold value may be a reliable source of information to classify patient with failed back surgery syndrome (FBSS) eligible for spinal cord stimulation (SCS) treatment.

A non-FFBS-affected area can be any area where the FFBS neuropathic effects are not present, such as, for example, the superior limb. It is noted that the average threshold value for the lowest electrical intensity sensitive to the user of a non-FFBS-affected area may be obtained using the same methods described herein for the average threshold value for the lowest electrical intensity felt by the user for the FFBS-affected areas, i.e. the buttock, the lumbar, the thigh and/or the calf; by taking the steps a) to c) with regard to the non-FFBS-affected area. It is further noted that therefore the data needed for obtaining the average threshold value of a non-FFBS-affected area could be obtained using a device according to any of the embodiments disclosed herein.

In some embodiments, the method further comprises the step of:
f) generating data for classifying patients with FBSS eligible for SCS treatment from the determined CPT wherein the data indicates one or more of the following:
- the threshold value as a function of stimuli frequency for the plurality of electrode pair positions; and
- one or more maps of the threshold value as a function of electrode pair position, each map indicating the threshold value for a single frequency.

It is noted that the data generated may help to enhance the reading and interpretation of the data when displayed to a user. For example, the threshold value of different electrode pair locations can be compared across different locations for a selection of frequencies or the threshold value of different electrode pair locations can be spatially displayed to enhance the spatial analysis of such threshold values.

Another computer-implemented method according to one or more embodiments, relates to a computer-implemented method to prognosticate the outcome of a patient with failed back surgery syndrome (FBSS) undergoing a spinal cord stimulation (SCS) treatment, wherein the method comprises the steps of any of the previous methods and further comprises the steps:
g) comparing the generated data with one or more standard values for the data; and
h) determining the outcome of the patient undergoing an SCS treatment from the significant deviations found in the comparison.

The standard values for the data may be obtained from the comparison of the threshold values for patients wherein the SCS treatment has been successful versus those whose SCE treatment was not successful, for a particular apparatus setup. Therefore, the standard values may be, for example, a threshold value that may serve as a reference to find a deviation with, in step h) (e.g. if the calculated threshold value is above the standard value then it is determined that there is a significant deviation).

A third aspect of the invention refers to computer program product comprising instructions which, when the program is executed by a processor, cause the computer to carry out the steps of any of the methods according to the second aspect of the invention. The computer program product may be implemented on hardware and/or software.

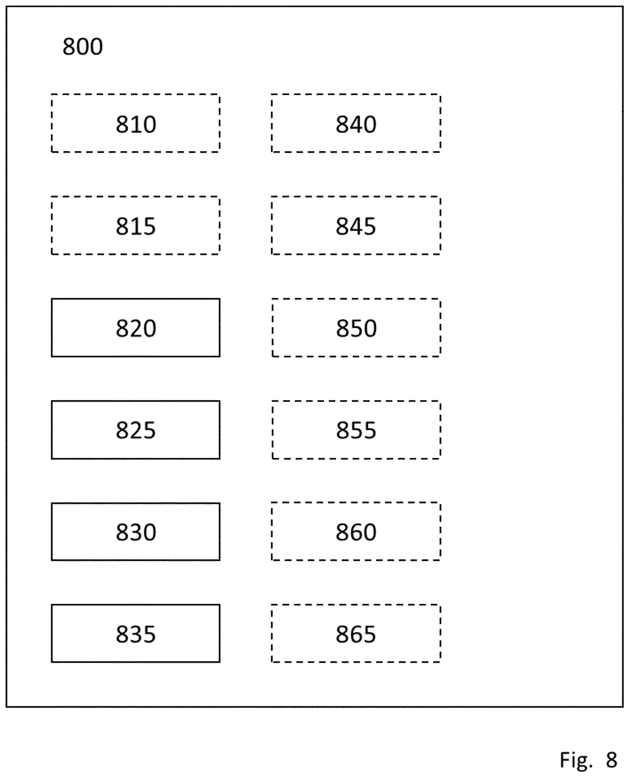

Figure 8 shows a computer program product 800 to implement the methods disclosed in the present document, that may be implemented in software, hardware or a combination thereof. The module may be saved in a memory of the system (for example, in the stimulator or the user interface), or may be saved remotely, for example, in a remote server or distributed computing system communicatively connected to the system. The computer program product 800 is communicatively connected to the electronic components of the system. The computer program product 800 may be configured to perform any of the steps of the method described with reference to Figures 3-7.

In some embodiments, the module may comprise an electrode pair selecting module 810 configured to select the electrode pair from a plurality of electrode pairs. In embodiments where a reference range for the intensities is to be determined, the electrode pair selecting module 810 is also configured to select the reference electrode pair to perform the steps to determine the reference range of electrical intensity. In embodiments where a reference range for the intensities is to be determined the electrode pair selecting module 810 is configured to select one of the electrodes for determining the reference rage (for example randomly). The electrode pair selecting module 810 is configured to select an electrode pair for each electrical stimulus to be provided by the system 10. The electrode pair selecting module 810 may be configured to ensure all the electrode pair positions have been provided with the multiple stimuli according to the method. In some embodiments the electrode pair selecting module 810 may therefore control an electrical switch or other electronic controller comprised in the stimulator or external to the stimulator to control which electrode pair each stimulus is sent. In some embodiments, the electrode pair selecting module 810 may be configured to receive a manual selection or deselection from the user via a user interface, so that the module 810 will ensure that electrical stimuli according to the method are or are not sent to the selected/deselected electrode pair indicated by the user via the user interface. The module 800 may select the electrode pairs according to a predetermined sequence saved in the memory. In the embodiments wherein the electrode pair selecting module 810 is not comprised in the module 800, a predetermined set of selected set of electrode pairs, may be used. In embodiments where the system 10 only comprises one electrode pair, the module 810 is not provided.

In some embodiments, the module may comprise a frequency selecting module 815 configured to select the frequency from a plurality of frequencies. In embodiments where a reference range for the intensities is to be determined the frequency selecting module 815 is configured to select the frequency from a plurality of frequencies for the reference stimuli when a reference range of electrical intensity is being determined. The frequency selecting module 815 is configured to select the frequency of each stimuli and control the stimulator 12 to provide the stimuli at the selected frequency. The frequency selecting module 815 may be configured to determine whether stimuli over all the frequencies have been provided according to the methods disclosed herein. In some embodiments, the frequency selecting module 815 may be configured to receive a manual selection or deselection of frequencies from the user via a user interface, so that the module 815 will ensure stimuli are/are not provided at the frequencies selected/deselected by the user via the user interface. In the embodiments wherein the frequency selecting module 815 is not comprised in the module 800, a predetermined set of selected frequencies may be used. In some embodiments only a single frequency may be used, in which case the module 815 may not be provided.

The module 800 further comprises an intensity selecting module 830 configured to select the intensity of the stimuli. The intensity selecting module 830 is configured to select the intensity of the stimuli applied when a threshold value is being determined. The intensity module is connected to the stimulator of the system to control the intensity to be provided. In some embodiments, the intensity selecting module 830 is configured to ensure all the intensities from a plurality of intensities are being provided. In some embodiments, the intensity selecting module 830 may be configured to receive the plurality of intensities from a user via a user interface.

Optionally, the intensity selecting module 830 is configured to select the intensity from a plurality of frequencies in the embodiments wherein a reference range of electrical intensity is being determined. It may be therefore configured to apply the intensities sequentially ascending and/or sequentially descending to find the reference range of electrical intensity. It may be configured to repeat a set of intensities from the plurality of intensities if the reference range of electrical intensity is too big unless it is within a desired range or a predetermined maximum number of iterations are performed.

The module comprises a stimulating module 820 configured to control, for the electrode pairs selected by the electrode pair selecting module 810,, the stimulator 12 to provide the stimuli with the frequencies selected by the frequency selecting module 815 and the intensity selected by the intensity selecting module 830 according to the invention. In embodiments where a reference range for the intensities is to be determined the stimulating module 820 is configured to provide control signals to the stimulator 12 to provide the electrical stimuli for determining the reference range.

The module further comprises an indication module 825 configured to determine if each stimulus has been felt by the user. The indication module 825 is connected to the feedback element of the system. The output of the feedback element indicates whether the stimulus has been felt. Thus, the indication module 825 is configured to determine if one or more stimulus have been felt by the user considering the output of the feedback element. For example, if the feedback element only provides indication if the user has felt the stimulus, the indication module 825 may determine the lack of output from the feedback element when a stimulus is provided as an indication of the stimulus not being felt, and an output coincident with a stimulus as an indication of the stimulus being felt. If the indication module 825 determines that none of the stimuli were felt for a given frequency, the indication module 825 instructs stimulating module 820 to provide further stimuli of higher intensities. If the indication module determines that all the stimuli were felt, the indication module 825 instructs the stimulating module 820 to provide further stimuli of lower intensities

Optionally, the stimulating module 820 is configured to interact with the indication module 825 to see if none or all stimuli were felt and if so, provides further stimuli. If the indication module 825 indicates that none of the stimuli were felt, the further stimuli are provided with higher intensities, while if all the stimuli were felt, the further stimuli are provided with lower intensities.

The module further comprises a threshold module 835, configured to determine, for the electrode pairs selected and the frequencies selected, the threshold based on the information provided by the indicating module 825 and the intensity selecting module 830. Given a set of intensities selected by the intensity selecting module 830 for a given electrode pair and frequency, the threshold module 835 will select as the threshold value the lowest electrical intensity the indication module 825 has determined as felt by the user. It is noted that any other criteria to select a threshold value may be used. For example, it may be selected as the threshold value the lowest intensity wherein at least the immediately higher intensity selected by the intensity selecting module 830 has been also determined by the indication module 825 as felt by the user. As another example, the threshold module 835 may require that an intensity has been determined by the indication module 825 as felt by the user at least twice or more than half of the times before being selected as the threshold value. The threshold module would be then configured to avoid false positive or phantom sensations from the user.

In some embodiments, the module may comprise an intensity range determination module 840, configured to determine a range of intensities from the information provided by the indication module 825 and the intensity selecting module 830 in the embodiments wherein a reference range is determined. If for an intensity selected by the intensity selecting module 830, the indication module 825 indicates the user has felt the stimuli, such stimulus is tagged as felt, and vice versa. Given a set of stimuli tagged, the intensity range determination module 840 can determine the intensity at which a change in the information provided by the indication module is noted. Such change may be determined as a limit (either upper or lower) of the range of electrical intensity. The intensity range determination module 840 may be configured to indicate the intensity selecting module 830 the range within the intensities may be selected from. In the embodiments wherein the intensity range determination module 840 is not comprised in the module 800, a predetermined set of intensities may be used.

In some embodiments, the module may comprise a standardisation module 845 configured to compare the average threshold value of the buttock, the lumbar, the thigh and/or the calf with the average threshold value of a non-FBSS-affected area of the body, like the upper limb. The standardisation module 845 is configured to compute a standardised value indicative of the difference between the average threshold value of the buttock, lumbar, thigh and/or calf and the threshold value of a non-FBSS-affected area of the body. The module 845 may in some embodiments calculate the ratio of the average threshold value the buttock, the lumbar, the thigh and/or the calf with respect to the average threshold value of a non-FBSS-affected area of the body, obtaining a standardised average threshold value of the buttock, the lumbar, the thigh and/or the calf.

In some embodiments wherein the system comprises a memory and the processor is configured to store, the module may comprise a data storage module 850, configured to store the information provided by the electrode pair selecting module 810, the frequency selecting module 815, the indication module 825, intensity selecting module 830, the threshold module 835, the intensity range determination module 840 and/or the standardisation module 845. The data storage module 850 may be configured to take information from these modules and save it to the memory, and optionally to further retrieve information from the memory and provide it back to these modules. Therefore, the data storage module 850 may be configured to store in the memory the electrode pair location selected from the electrode pair selecting module 810, the frequency from the frequency selecting module 815 and/or the intensity from the intensity selecting module 830, regarding the stimuli provided to the user. The data storage module 850 may be further configured to store in the memory the indications whether the stimuli was felt but the user from the indication module 825. The data storage module 850 may be further configured to store in the memory the average threshold value determined by the threshold module 835. The data storage module 850 may be further configured to store in the memory the intensity ranges indicated by the intensity range determination module 840. The data storage module 850 may be further configured to store in the memory the standardised average threshold value determined by the standardisation module 845. It is noted that the data storage module may store any other information in the memory. In the embodiments wherein the electrode pair selecting module 810, the frequency selecting module 815 and/or the intensity range determination module 840 are not comprised in the module 800, the a data storage module 850 may save the related information from a predetermined set of selected set of electrode pairs, frequencies and/or intensities.

In some embodiments wherein the system is configured to prognosticate the outcome of a patient with failed back surgery syndrome (FBSS) undergoing a spinal cord stimulation (SCS) treatment, the module comprises a comparison module 855 configured to compare an average threshold value with a predetermined standard average threshold value. The comparison module 855 may receive information from the data storage module comprising the average threshold value and/or the standardised average threshold value. Then the information of the comparison will be provided to the outcome module 860. It is noted that the comparison may be stored in the memory by the data storage module 850.

In some embodiments wherein the system is configured to prognosticate the outcome of a patient with FBSS undergoing an SCS treatment, the module comprises an outcome module 860, configured to determine an outcome base on the comparison information provided by the comparison module 855. The outcome might comprise the response of the patient to the SCS treatment, a certainty level of such response as well as any other information that could help take a decision on the SCS treatment, like information regarding the mental health condition of the user. It is noted that the outcome may be stored in the memory by the data storage module 850.

In some embodiments, wherein the system further comprises a display device to show the data, the module comprises a display module 865, configured to display the data generated by the processor, Therefore, the display module 865 may be configured to display in the display device the electrode pair location selected from the electrode pair selecting module 810, the frequency from the frequency selecting module 815 and/or the intensity from the intensity selecting module 830, regarding the stimuli provided to the user. The display module 865 may be further configured to display in the display device the indications whether the stimuli was felt but the user from the indication module 825. The display module 865 may be further configured to display in the display device the average threshold value determined by the threshold module 835. The display module 865 may be further configured to display in the display device the intensity range indicated from the intensity range determination module 840. The display module 865 may be further configured to display in the display device the standardised average threshold value determined by the standardisation module 845. The display module 865 may be configured to display in the display device the comparison from the comparison module 855. The display module 865 may be configured to display in the display device the outcome from the outcome module 860. The display module 865 may be further configured to display in the display device any of the previous information from the data storage module 850. It is noted that the display module 865 may display the information in preferred ways to enhance the reading and interpretation of the data displayed. In some embodiments, the data may be displayed in the display device in form of a lookup table. In some embodiments, the threshold value and/or the average threshold value may be displayed as a function of stimuli frequency for the plurality of electrode pair positions. In some embodiments, one or more maps of the threshold value as a function of electrode pair position may be displayed, each map indicating the threshold value for a single frequency.

### EXAMPLE

A study was conducted to quantify the current perception thresholds (CPT) of the patient using electrical stimuli of different frequencies and current amplitudes at different electrode pair locations.

### Materials and method

### Patients

For the study, a total of 33 patients diagnosed with FBSS and potential candidates for the trial period with an SCS device were sequentially recruited. After a multidisciplinary assessment based on the patient's medical history, physical examination, biopsychosocial assessment, and neuroimaging studies, patients were classified as surgery candidate (SC) or not surgery candidate (NSC) for SCS trial. 18 patients were included in the SC group and 15 were included in the NSC group. In a separate session, the patients who agreed to participate in the study were evaluated to collect their CPT with a system as described herein.

Inclusion criteria were patients older than 18 years, diagnosed with FBSS who presented symptoms of low back pain and/or pain of neuropathic origin in the lower limbs, that were refractory to conservative analgesic treatments, with more than three months of evolution, presented pain intensity, measured with the Visual Analogical Scale (VAS), greater or equal than 5, not candidate for new spinal surgery and had an adequate understanding of the risks and benefits of treatment with SCS. Exclusion criteria were previous spinal interventions with a time of evolution of less than 6 months, vertebral instability with risk of progression, pregnancy, carriers of pacemaker and/or defibrillator (contraindication to use the EQSA system), the presence of tumours in the area of application of the EQSA system or allergies to the electrodes of the EQSA.

### SCS Trial

For the patients undergoing the SCS trial, the Exclusion criteria were: 1. Present behavioural disorders related to opioid use that suggest abuse or playful use of opioids (e.g., escalating doses without a prescription, loss of prescriptions, loss of opioid medications, obtaining opioids from multiple prescribers); 2. Present psychological or psychiatric comorbidities that contraindicate the implant (e.g., untreated high mood disorders, untreated psychosis, severe personality disorders); 3.Present coagulopathies, immunosuppression, local or systemic infections; 4.Present any associated pathology that conditions an unacceptable surgical risk; 5.The inability of the patient to understand the technique and/or use of neurostimulation. The SCS trial was finally performed in 15 patients in the SC group. In other three SC group patients, the SCS trial was delayed because the presence of intercurrent disease in one case, and by choice of the patients in the other two

The SCS trial phase lasted 8 days. The therapy consisted of implanting two octopolar cylindrical electrodes percutaneously (Vectris SureScan, Minneapolis, MN) in the posterior epidural space at thoracic level (Day 0). Access was made with fluoroscopy support, at paraspinal level and the T12-L1 or L1-L2 vertebral segments were selected, depending on the patient's anatomy. The final location of the electrode was determined intraoperatively, after achieving that the paraesthesia caused by the neurostimulation covered all the painful areas referred by the patient. The distal end of the electrode must be placed over the T8/T9 segments. Once implanted, during the entire test phase, the electrodes were connected to an external neurostimulator.

All patients, during the trial period, received sequential treatment with the two stimulation patterns commonly used in daily clinical practice: a. Low-dose stimulation (LD), with frequencies 40- 60 Hz, pulse widths 210-400 µsec and a current amplitude which the patient reported as feeling paraesthesia in the painful area; b. High-dose stimulation (HD) with frequencies 1,000 Hz, pulse widths 210 µsec, and amplitudes 1-5 mA. Patients received the first stimulation with HD for 4 days (Day 1 to 4), after which they will start the stimulation with the LD alternative pattern, for another 4 days (Day 5 to 8). On the 5th day, whether or not the patient has responded positively to the initial therapy, the change in the stimulation programming was made to initiate the alternative pattern.

Nine days after the implantation of the electrodes, the definitive evaluation of the analgesic benefit obtained with SCS was made. If the analgesic benefit, measured by the VAS and the percentage of global improvement at the back and/or leg, was equal or superior to 50% of the basal pain, it was considered as "responsive" and the internal pulse generator (IPG) was implanted to complete the system implantation. If the benefit was less, the patient was considered "non-responsive" to SCS therapy, and the electrodes were explanted. The implantation of the IPG (Intellis or Restore; Minneapolis, MN) was performed at a subcutaneous level in the upper part of the left buttock and connected to the proximal end of the electrodes. After the implant, the IPG was programmed with the pattern with which the patient has felt most comfortable during the trial period (HD or LD).

Respondent patients received follow-up visits at one week, fifteen days, one month and three months after the neurostimulator was implanted. During the follow-up period, patients were reevaluated by means of various questionnaires, in order to find out the evolution of functionality, quality of life, degree of disability, pain intensity and level of satisfaction with the therapy. During the clinical study, possible adverse events were collected systematically.

### Assessment of pain, quality of life, disability and mental health

Several questionnaires were used to analyse the following variables: 1.Pain assessment: Pain intensity (VAS) and Neuropathic pain diagnostic questionnaire (DN4); 2. Quality of life health-related: The 36-Item short-form health survey (SF-36v1)^{©}; 3. Disability: Oswestry Disability Index (ODI); 5. Mental disorders: Hospital Anxiety and Depression Scale (HADS), Reduced Pain Coping Questionnaire (CAD-R), and Beck Depression Inventory (BDI-II)^{©}. A small explanation on each is not provided:
1. Pain assessment

The Visual Analogical Scale (VAS) consists of a 10 cm line representing the continuous spectrum of painful experience. At its left end or bottom is described "no pain" and at its right end or top, "maximum pain or the worst pain imaginable", with no other description along the line. It contains no numbers or descriptive words. VAS was used for measures independently back (VAS Lumbar) and inferior limb pain (VAS Irradiated).

The Neuropathic pain diagnostic questionnaire (DN4) consists of four questions with 10 items at all, 7 related to symptoms and 3 to physical examination. The 3 physical examination items consist in explore the presence of hypoesthesia to touch, hypoesthesia to pinprick, and pain with brushing (Hyperesthesia/allodynia), realized in our case with a cotton, a sharp wooden toothpick, and a soft brush, respectively. The location indicated by the patient as the one with maximum pain were compared with the mirrored location on the contra lateral side. If the pain was bilateral a location without pain as close as possible to the original mirror site was tested for comparison. The ten items are evaluated with 1 (yes) or 0 (no). A score ≥4 is considered practically pathognomonic of neuropathic pain, with a sensitivity of 82.9%, and a specificity of 89.9% .A validated Spanish version was used (Perez, 2007).

### 2. Quality of life health-related

The 36-Item Short-Form Health Survey (SF-36v1)^{©}, is a self-administered questionnaire, which assesses health-related quality of life (HRQoL) in the general population and in specific subgroups (Ware 1992; Ware 2000). It has 36 items that measure eight domains of health status: physical functioning; role limitations because of physical problems; bodily pain; general health perceptions; vitality; social functioning; role limitations because of emotional problems; and mental health. For each domain, scores are coded, summed, and transformed to a continuous scale from 0 to 100, with higher scores indicating better health. The eight areas combine two final and general scores: The physical component summary (PCS) and mental component summary (MCS). PCS and MCS were standardized by linearly transform to the T-score metric, which has a mean of 50 and a standard deviation of 10 for the Spanish general population (Vilagut 2008). This standardization offers the advantage of providing a direct interpretation of the scores relative to general population. The calculation process for the eight domains, PCS and MCS was done using a specific software provided by QualityMetric, Inc. The validated Spanish-translated version was used (Alonso 1995, Alonso 1998).

### 3. Disability

The Oswestry Disability Index (ODI) is a self-report questionnaire developed to assess the degree of functional disability related to low back pain (Fairbank, 1980). It contains ten sections relating to activities of daily living: intensity of pain, lifting, self-care, ability to walk, ability to sit, sexual function, ability to stand, social life, sleep quality, and ability to travel. Each question is scored on a scale of 0-5, where no difficulty = 0 and maximum difficulty = 5. The total of these is multiplied by 2 to give a score from 0 to 100. The scores categories are: 0-20 minimal disability, 21-40 moderate disability, 41-60 severe disability, and 61-80 crippling back pain. A rate of 81-100 may represent an exaggeration of symptoms and should be carefully evaluated. The validated Spanish version was used (Florez Garcia 1995; Selva-Sevilla 2019).

### 4. Mental Disorders

The Hospital Anxiety and Depression Scale (HADS), is a self-assessment instrument for detecting anxiety and depression, being used both for diagnosis and to assess the severity of the disorder. It is composed of 14 items, divided into 2 subscales: anxiety (HADS-A) and depression (HADS-D), with 7 items each. The HADS is scored by summing the ratings for the 14 items to yield a total score, and by summing the ratings for the seven specific items to yield separate scores for HADS-A and HADS-D. Each item on the questionnaire is scored from 0 to 3, so the total score ranges from 0 to 42, and for each of the subscales range from 0 to 21. Recommended cut-off scores are 8-10 for doubtful cases and ≥11 for definite cases (Zigmond 1983). Optimal balance between sensitivity and specificity was achieved when caseness was defined by a score of 8 or above on both HADS-A and HADS-D (Bjelland, 2002). The scores categories for HADS-A and HADS-D are: normal (0-7); mild (8-10); moderate (11-14); severe (15-21). We used the validated Spanish version (Tejero 1986; Herrero 2003; Quintana 2003).

The reduced form of the Pain Coping Questionnaire (CAD-R; In Spanish "Cuestionario de Afrontamiento del Dolor") is a reduced version of the original pain coping questionnaire (CAD), which that measure six different dimensions distributed in 31 items. The CAD-R maintained the original six dimensions, but reduced the length to 24 items and added two general second-order scales. There are four items for each dimension. Items are scored with a 5-point Likert scale (1 = never; 2= seldom; 3= sometimes; 4= often; 5= always). The first-order dimensions are religion (use of religious beliefs), catharsis (seeking social emotional support), distraction (avoidance by taking away attention from the pain), self-affirmation (not fainting, giving encouragement), mental self-control (mental control of pain) and information seeking (counselling, consultations on what to do). Dimensions are grouped into "passive" pain coping (Religion and catharsis), and "active" pain coping the remaining dimensions. Scores for each dimension can be calculated as the sum of the scores of the constituent items. The scores for the passive and active coping dimensions range from 8 to 40 and from 16 to 80, respectively. This instrument was originally constructed and validated in Spain (Soriano 2004; Cánovas 2018).

The Beck Depression Inventory second edition (BDI-II)^{©} is one of the most commonly used tools to measure the severity of a depression (Beck, 1961). It is a self-administered questionnaire that consists of 21 multiple choice questions, each composed of several statements about the same depressive symptom, ordered from least to most severe, and from which the person evaluated should choose the one that best describes his/her condition. In each of these items, the different statements are scored from 0 to 3, so the inventory score range is 0 to 63. There are cut-off scores that define different levels of severity of depressive symptomatology: 0-13 indicates minimal depression, 14-19 mild depression, 20-28 moderate depression and 29-63 severe depression. We used the validated version translated into Spanish (Conde, 1975).

Current perception thresholds (CPT)In this study a system as described herein was used to apply transcutaneous electrical stimulation to the back and legs of the patients to quantify the CPT. The stimulator was a portable electronic system able to generate a current-controlled square-shaped waveform at 5Hz, 250Hz and 2000Hz frequencies and up to 9mA with 0.1mA of resolution. The stimulator was designed to be used with multiple electrode pairs and control independently up to 48 stimulation channels, each one associated with an electrode pair. The multiple electrode pairs were responsible of transmitting the current generated by the stimulator to the patient. Four different multiple electrode pair arrays were designed specifically to cover different areas of the body: low back (11 electrode pairs), gluteus (9 electrode pairs), lateral thigh (13 electrode pairs) and lateral leg (7 electrode pairs). A fifth array (3 electrode pairs) was designed to serve as a reference array to be placed on a non-FBBS-affect area, which was placed on the arm and forearm.

The electrode pairs in all cases were designed as concentric pairs, formed by an inner circular electrode of 10mm of diameter and a ring electrode of 30mm inner diameter and 40mm outer diameter. These electrodes were always activated in pairs to delimit their activation area, ensuring that the current would just travel from the outer ring to the inner circle or vice versa. Finally, a custom-designed software application running in a laptop allowed the clinicians to supervise and configure the sensory analysis carried out by the system and to store the results of the analysis. A commercial hand-held switch (CompuPhase - Netherlands) was connected to the PC via USB and the system was controlled by means of Bluetooth communication.

For each electrode pair and each frequency, the system would follow a semi-automatic procedure to gather the CPT. The patient was instructed to push the switch every time a stimulus was felt during the full procedure, so the EQSA system could register if each stimulus was felt or not by the patient. Furthermore, the software application was kept out of the sight of the patient to ensure that no clue about the timing or type of stimuli was provided.

First, the clinician would select an electrode pair as a reference, for defining the initial intensity ranges of the patient. Then the EQSA would apply a ramp starting at 0mA, with ascending steps of 0.5mA, stimuli of 2s of duration and 2s of rest between them, until the patient referred any sensation. After that, a descending ramp was applied, starting 0.5mA higher than the intensity at which the patient referred a sensation, with descending steps of 0.5mA, stimuli of 2s of duration and 2s of rest between them, until the patient stopped referring any sensation. This procedure was repeated 3 times and the initial intensity ranges were defined as the minimum intensity at which a stimulus was felt and the maximum intensity at which a stimulus was not felt.

Once the initial intensity ranges were defined for that patient, the following procedure was applied for each concentric electrode pair. A random electrode pair was automatically selected, and a random intensity was applied during 2s of duration followed by a rest of between 2 and 4 seconds of duration. This was repeated for each electrode pair with all the intensities (at random order) comprised inside the initial intensity ranges with a resolution of 0.05mA. The CPT of each electrode pair and frequency was finally determined as the minimum intensity at which a stimulus was felt, and the two consecutive intensities were felt as well.

The exploration was performed in a quiet room to avoid distraction at stable temperature of 25°C. For testing patients lay in bed in decubitus lateral over the less symptomatic side with a pillow between the legs. It was of interest to explore the most frequent painful areas in patients with FBSS. The testing areas were the low back, the more symptomatic lower limb (gluteous region, lateral border of thigh and lateral border of leg) and the ipsilateral upper extremity as non-affect body-region and reference (proximal and distal third of the lateral aspect of the arm and proximal third of the anterior aspect of the forearm). Electrodes were placed only on intact skin, cleaned with alcohol wipes (preinjection swabs) and allowed to dry. The patient assessment took approximately 2hr.

### Statistical methods

Patients were classified according to whether they were candidates for surgery (surgery candidates or SC) or not (non-surgery candidate or NSC). The categorical variables under study are presented as absolute frequency and percentage, and the quantitative variables as mean and standard deviation. Differences between the two analysis groups were evaluated using nonparametric Mann-Whitney U test and the Chi-square test, as appropriate.

Current Perception Thresholds (CPTs) or sensitive thresholds were evaluated through centralization summary statistics: absolute average (mean electrode value) and standardized average (ratio of the mean electrode value and the mean value of the reference electrode). The existence of differences in sensitive thresholds between the two groups was studied by using the nonparametric Mann-Whitney U test. To evaluate the association of the sensitive thresholds with the scales of patient's pain assessment (VAS), disability (Oswestry), quality of life (SF-36) and psychological situation (HADD and BDI-BECK), Spearman's nonparametric correlation coefficient was calculated. All analyses were stratified according to electrode region (buttock, lumbar, thigh, calf and reference) and frequency (5 Hz, 250 Hz, 2 kHz).

The clinical improvement of patients with definitive implants 3 months after surgery was independently and blindly assessed by five expert clinicians based on the results of the EDS, DN4, HAD-S, BDI-II, ODI, EVA and SF36 questionnaires. The following improvement categories were considered: excellent, very good, good, moderate, and poor. The degree of inter-observer agreement was analysed using the weighted kappa coefficient. The average sensitive thresholds of the group excellent was compared to the remaining using Mann-Whitney U tests. A sensitivity analysis evaluated the impact of comparing the group of patients with excellent or very good improvement vs others.

### Results

The SCS trial was successful (VAS and the percentage of global improvement at the back and/or leg, was equal or superior to 50% of the baseline pain) in all 15 patients, and the IPG implant was performed in all of them. Finally, the follow-up period was completed by 14 patients, because one of them suffered an implant infection and the neurostimulation system was explanted.

### Clinical characteristics

The baseline characteristics of the patients according to the decision to undergo surgery or not are shown in Table 1. Significant differences were found in the BDI-II (SC group: mean 17.2 (SD 9.7); NSC group: 23.9 (9.0); p-value=0.04) and in the SF36-MCS (SC group: 45.5 (10.7); NSC group: 32.2 (6.7), p-value<0.01) questionnaires.

Although the differences in the levels of anxiety and depression measured by the HADS questionnaire were not statistically significant, they were clinically relevant. Thereby, while the patients in the SC group presented mild levels in the scores categories of anxiety and depression (HADS-A: 9.6 [4.4]; HADS-D: 8.1[4.5]), those in the NSC group presented moderate levels in both cases (HADS-A: 11.5 [3.0]; HADS-D: 10.6 [3.4]).

In addition, significant differences were found in the variable time in years with pain (SC group: 6.5 (3.5); NSC group; 9.9 (5.2), p-value=0.01) and in the use of opioids (SC group: 16 (89%); NSC group: 8 (53%), p-value=0.02), but not with the others drugs.

Finally, there were no differences in age, gender, incidence of neuropathic pain, pain intensity, disability and number and type of surgeries between groups.

**Table 1: Patients' clinical baseline characteristics**

| **Variables** | **NSC N=15 Mean (SD)** | **SC N=18 Mean (SD)** | **P-value** |
|---|---|---|---|
| **Age** | 50.1 (8.5) | 54.0 (11.3) | 0.33 |
| **Gender** | | | |
| Female | 12 (80%) | 11 (61%) | 0.24 |
| Male | 3 (20%) | 7 (39%) | |
| **Visual analog scale (VAS)** | | | |
| VAS irradiated | 6.7 (1.1) | 6.2 (2.5) | 0.81 |
| VAS lumbar | 7.0 (1.4) | 7.5 (1.4) | 0.34 |
| **Hospital Anxiety and Depression Scale (HADS)** | 22.1 (5.1) | 17.7 (8.4) | 0.13 |
| Hospital Depression (HADS-D) | 10.6 (3.4) | 8.1 (4.5) | 0.16 |
| Hospital Anxiety (HADS-A) | 11.5 (3.0) | 9.6 (4.4) | 0.14 |
| **Beck Depression Inventory (BDI-II)** | 23.9 (9.0) | 17.2 (9.7) | 0.04* |
| **Health Survey Questionnaire Short Form 36 (SF-36)** | | | |
| Physical function (SF36-PCS) | 28.9 (9.1) | 29.7 (78) | 0.73 |
| Mental function (SF36-MCS) | 32.2 (6.7) | 45.5 (10.7) | <0.01** |
| **Oswestry Disability Index (ODI)** | 55.2 (12.6) | 53.2 (13.4) | 0.70 |
| **Reduced Pain Coping Questionnaire (CAD-R)** | | | |
| CAD-R: Distraction | 9.8 (3.9) | 10.6 (3.6) | 0.48 |
| CAD-R: Information seeking | 9.7 (4.0) | 10.8 (3.5) | 0.31 |
| CAD-R: Use of religious beliefs | 7.6 (5.1) | 7.4 (4.3) | 0.86 |
| CAD-R: Catharsis | 8.9 (3.3) | 8.4 (4.0) | 0.57 |
| CAD-R: Self-control | 10.1 (3.7) | 11.2 (4.5) | 0.44 |
| CAD-R: Self-affirmation | 14.9 (3.5) | 14.8 (4.9) | 0.77 |
| CAD-R: Active pain coping | 44.5 (8.4) | 47.4 (11.1) | 0.41 |
| CAD-R: Passive pain coping | 16.5 (6.2) | 15.9 (7.1) | 0.80 |
| **Neuropathic pain diagnostic (DN4)** | 6.6 (1.5) | 6.4 (1.5) | 1.00 |
| Hypoesthesia's n (%) | 14 (93%) | 15 (83 %) | 0.38 |
| Hyperesthesia's n (%) | 5 (33%) | 7 (39 %) | 0.74 |
| **Time with pain in years** | 9.9 (5.2) | 6.5 (3.5) | 0.01* |
| **Lumbar pain** | 15 (100%) | 18 (100%) | - |
| **Leg pain** | | | |
| No pain | 0 (0%) | 1 (6%) | 0.39 |
| Unilateral pain | 8(53%) | 12 (67%) | |
| Bilateral pain | 7 (47%) | 5 (28%) | |
| **Number of surgeries with instrumentation** | | | |
| 0 | 0 (0%) | 1 (6%) | 0.14 |
| 1 | 8(53%) | 14 (78%) | |
| 2 | 7 (47%) | 3 (17%) | |
| Drugs | | | |
| Antidepressant | | | |
| No | 8 (53%) | 9 (50%) | 0.85 |
| Yes | 7 (47%) | 9 (50%) | |
| Anti-epileptic | | | |
| No | 11 (73%) | 10 (56%) | 0.29 |
| Yes | 4 (27%) | 8 (44%) | |
| Opioids | | | |
| No | 7 (47%) | 2 (11%) | 0.02* |
| Yes | 8(53%) | 16 (89%) | |
| Benzodiazepines | | | |
| No | 9 (60%) | 10 (56%) | 0.80 |
| Yes | 6 (40%) | 8 (44%) | |
| Minor analgesics /Others | | | |
| No | 1 (7%) | 0 (0%) | 0.27 |
| Yes | 14 (93%) | 18 (100%) | |

### CPTs

The comparison of CPTs values of SC and NSC groups is shown in Table 2.

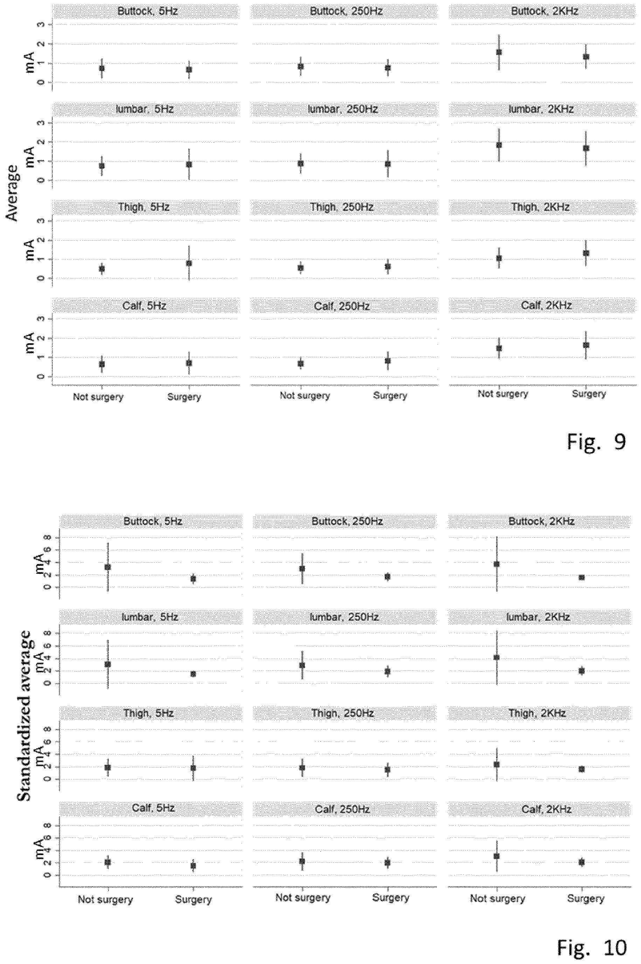

In the NSC group there was a general trend showing higher values in the standardized threshold in all the zones and frequencies explored and lower in the average values at the reference zone, although these differences did not reach statistical significance (Figure 9 and Figure 10). This trend was particularly relevant at 5Hz frequency, both in the average threshold at the unaffected reference region (mean [SD] 0.33 [0.19] vs 0.63 [0.75], p-value= 0.08) and in the standardized thresholds at the buttock (3.21 [3.95] vs 1.36 [0.83], p-value= 0.09, for NSC and SC respectively) and the lumbar region (3.06 [3.92] vs 1.50 [0.51], p-value= 0.09) for NSC and SC groups respectively.

In the physical examination, there were no significant differences in the degree of presentation of hyperesthesia (33% vs 39% in NSC and SC groups, respectively) and hypoesthesia (93% vs 83% in NSC and SC groups, respectively). Patients with hypoesthesia showed higher thresholds than hyperesthesia patients in all the explored territories, including the arm (p <0.05 in all comparisons, data not shown).

**Table 2: Sensitivity thresholds mean and standard deviation for each location tasted and both as an absolute value and as a standardized averaged with respect to the reference location.**

| **Variables** | **NSC N=15 Mean (SD)** | **SC N=18 Mean (SD)** | **P-value** |
|---|---|---|---|
| AVERAGE | | | |
| Buttock | | | |
| 5 Hz | 0.72 (0.52) | 0.64 (0.47) | 0.63 |
| 250 Hz | 0.82 (0.50) | 0.75 (0.44) | 0.96 |
| 2 kHz | 1.56 (0.93) | 1.33 (0.64) | 0.71 |
| Lumbar | | | |
| 5 Hz | 0.74 (0.52) | 0.83 (0.83) | 0.95 |
| 250 Hz | 0.87 (0.53) | 0.86 (0.72) | 0.74 |
| 2 kHz | 1.83 (0.87) | 1.67 (0.90) | 0.56 |
| Thigh | | | |
| 5 Hz | 0.50 (0.31) | 0.79 (0.90) | 0.31 |
| 250 Hz | 0.55 (0.33) | 0.61 (0.39) | 0.42 |
| 2 kHz | 1.05 (0.54) | 1.31 (0.68) | 0.43 |
| Calf | | | |
| 5 Hz | 0.63 (0.45) | 0.70 (0.58) | 0.40 |
| 250 Hz | 0.68 (0.32) | 0.82 (0.48) | 0.40 |
| 2 kHz | 1.47 (0.55) | 1.63 (0.74) | 0.66 |
| Reference | | | |
| 5 Hz | 0.33 (0.19) | 0.63 (0.75) | 0.08 |
| 250 Hz | 0.36 (0.19) | 0.56 (0.66) | 0.39 |
| 2 kHz | 0.70 (0.39) | 0.93 (0.63) | 0.27 |
| | | | |
| STANDARDIZED AVERAGE | | | |
| Buttock | | | |
| 5 Hz | 3.21 (3.95) | 1.36 (0.83) | 0.09 |
| 250 Hz | 2.95 (2.48) | 1.71 (0.69) | 0.35 |
| 2 kHz | 3.71 (4.43) | 1.56 (0.44) | 0.40 |
| Lumbar | | | |
| 5 Hz | 3.06 (3.92) | 1.50 (0.51) | 0.09 |
| 250 Hz | 2.82 (2.27) | 1.89 (0.91) | 0.10 |
| 2 kHz | 4.12 (4.35) | 2.00 (0.75) | 0.38 |
| Thigh, | | | |
| 5 Hz | 1.87 (1.40) | 1.76 (2.00) | 0.45 |
| 250 Hz | 1.81 (1.45) | 1.52 (1.14) | 0.90 |
| 2 kHz | 2.33 (2.68) | 1.60 (0.51) | 0.42 |
| Calf | | | |
| 5 Hz | 2.11 (1.09) | 1.52 (1.05) | 0.11 |
| 250 Hz | 2.22 (1.47) | 2.00 (0.92) | 0.96 |
| 2 kHz | 3.04 (2.51) | 2.05 (0.78) | 0.65 |

### Correlations between CPTs and Clinical measures

The results of the correlation analysis are summarized in Table 3. The average threshold presented significant negative correlations with the physical health assessed with the SF36-PCS (i.e. The higher the CPTs, the worse is the physical health). This negative correlation is conserved practically in all the zones and frequencies explored including the non-affect body-region zone at 5Hz (p<0.05 or p<0.01 in all the cases, except lumbar region at 250Hz, and the reference zone at 250Hz and 2 KHz).

There were also positive correlations between the average CPTs and the irradiated VAS in all areas explored except the calf, and with the disability (ODI) at the buttock (i.e. The higher the CPTs, the greater the pain and disability).

Mental health assessed with the SF36 questionnaire (SF36-MCS), was negatively associated with the standardized average in all the explored regions at 5 Hz (p<0.05, in all cases) (i.e. The greater the difference between the pathological zone explored and the reference zone, the worse is the mental health). Also, with the standardized average, a positive correlation where seen between lumbar CPTs at 5Hz and the depression (BDI-II) (p<0.05), and buttock CPTs at all the frequencies and disability (ODI) (p<0.05, in all cases).

**Table 3: Correlation between the CPT and the different questionnaires for each location tasted and both as an absolute value and as a standardized averaged with respect to the reference location. **p<0.01. *p<0.05.**

| | **VAS irradiated** | **VAS lumbar** | **ODI** | **SF36-PCS** | **SF36-MCS** | **HADS-A** | **HADS-D** | **BDI-II** |
|---|---|---|---|---|---|---|---|---|
| AVERAGE | | | | | | | | |
| Buttock | | | | | | | | |
| 5Hz | 0.19 | 0.17 | 0.45* | -0.38* | -0.21 | 0.16 | 0.19 | 0.16 |
| 250Hz | 0.40* | 0.19 | 0.34 | -0.36* | -0.11 | 0.18 | 0.15 | 0.09 |
| 2KHz | 0.30 | 0.29 | 0.39* | -0.47* | -0.15 | 0.07 | 0.08 | 0.07 |

| Lumbar | | | | | | | | |
|---|---|---|---|---|---|---|---|---|
| 5Hz | 0.53** | -0.05 | 0.10 | -0.37* | -0.14 | 0.22 | 0.20 | 0.08 |
| 250Hz | 0.36* | -0.03 | 0.08 | -0.17 | -0.20 | 0.14 | 0.11 | 0.06 |
| 2KHz | 0.35* | -0.03 | 0.01 | -0.34* | -0.10 | -0.01 | 0.00 | -0.07 |
| Thigh | | | | | | | | |
| 5Hz | 0.31 | 0.27 | 0.23 | -0.39* | -0.05 | 0.03 | 0.02 | -0.00 |
| 250Hz | 0.50** | 0.31 | 0.24 | -0.52** | -0.11 | 0.05 | 0.13 | 0.03 |
| 2KHz | 0.42* | 0.24 | 0.24 | -0.47* | -0.01 | 0.02 | 0.02 | -0.05 |

| Calf | | | | | | | | |
|---|---|---|---|---|---|---|---|---|
| 5Hz | 0.09 | 0.23 | 0.24 | -0.43* | -0.08 | 0.05 | 0.12 | 0.08 |
| 250Hz | 0.16 | 0.33 | 0.17 | -0.53** | -0.03 | 0.04 | 0.03 | 0.08 |
| 2KHz | 0.23 | 0.08 | 0.11 | -0.54** | -0.09 | -0.00 | 0.02 | -0.02 |

| Reference | | | | | | | | |
|---|---|---|---|---|---|---|---|---|
| 5Hz | 0.30 | 0.05 | -0.04 | -0.35* | -0.11 | 0.10 | 0.01 | 0.04 |
| 250Hz | 0.32 | -0.00 | -0.10 | -0.30 | -0.03 | 0.22 | 0.09 | 0.08 |
| 2KHz | 0.35* | 0.08 | -0.07 | -0.26 | -0.09 | 0.20 | 0.11 | 0.12 |

| STANDARIZED AVERAGE | | | | | | | | |
|---|---|---|---|---|---|---|---|---|
| Buttock | | | | | | | | |
| 5Hz | -0.24 | 0.05 | 0.39* | 0.07 | -0.33* | 0.00 | 0.15 | 0.25 |
| 250Hz | -0.17 | -0.04 | 0.37* | 0.07 | -0.20 | -0.08 | 0.01 | 0.12 |
| 2KHz | -0.14 | -0.08 | 0.41* | -0.17 | -0.17 | -0.18 | -0.13 | 0.04 |

| Lumbar | | | | | | | | |
|---|---|---|---|---|---|---|---|---|
| 5Hz | 0.20 | -0.18 | 0.18 | 0.00 | -0.37* | 0.33 | 0.33 | 0.36* |
| 250Hz | 0.00 | -0.10 | 0.17 | 0.13 | -0.37* | -0.14 | -0.04 | 0.14 |
| 2KHz | -0.05 | -0.15 | 0.02 | 0.04 | -0.15 | -0.33 | -0.25 | -0.18 |

| Thigh | | | | | | | | |
|---|---|---|---|---|---|---|---|---|
| 5Hz | -0.06 | 0.18 | 0.39* | -0.03 | -0.35* | -0.00 | 0.15 | 0.15 |
| 250Hz | 0.10 | 0.16 | 0.32 | -0.23 | -0.33 | -0.20 | 0.02 | 0.06 |
| 2KHz | 0.01 | 0.09 | 0.30 | -0.14 | -0.03 | -0.18 | -0.03 | -0.11 |

| Calf | | | | | | | | |
|---|---|---|---|---|---|---|---|---|
| 5Hz | -0.14 | 0.00 | 0.28 | -0.04 | -0.38* | -0.08 | 0.12 | 0.15 |
| 250Hz | -0.28 | 0.25 | 0.31 | -0.12 | -0.15 | -0.27 | -0.09 | 0.06 |
| 2KHz | -0.32 | -0.02 | 0.14 | -0.10 | -0.13 | -0.33 | -0.18 | -0.18 |

### CPTs in patients with SCS treatment

The clinical assessment of patients' improvement with SCS treatment is shown in Table 4. The comparison between the group that was considered to have an excellent response to treatment with the rest is shown in Table 5, and between the excellent and very good response group with the rest in Table 6.

Significant higher averages CPTs were seen in patients with an excellent response to SCS compared to the rest, in the lumbar region at 5Hz, 250Hz and 2KHz (p=0.01; p<0.01; and p=0.01, respectively) and the buttock region with the same frequencies (p=0.01; p=0.01; and p=0.04, respectively). Also, similar differences between both groups were seen at 5Hz frequency in the calf and the reference region (p=0.02 and p=0.05, respectively). With the standardized averages, only in the thigh at 2KHz were seen significant differences (p=0.05) but the other areas still exhibited somewhat substantial differences.

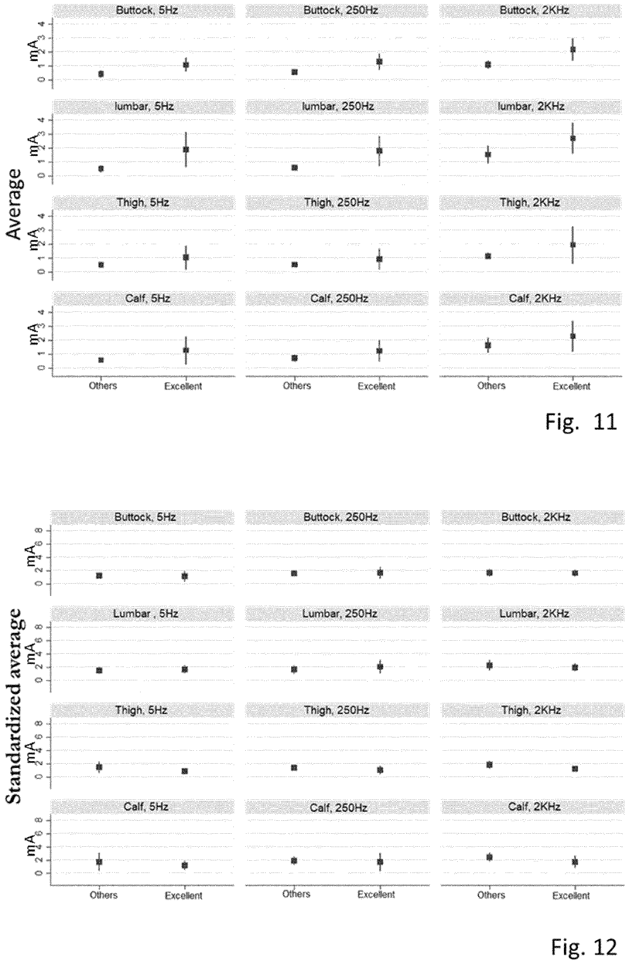

When the patients with excellent and very good responses were grouped and compared with the rest, they continued to observe higher averages CPTs in the better responses group, but only significant differences were seen in the lumbar region at 5Hz y 250Hz (p=0.02 in both cases) (Figure 11 and Figure 12).

**Table 4: Determination of improvement according to clinicians at three-month follow-up after SCS implant.**

| | **Frequency of categories** | **N** |
|---|---|---|
| 1.Excellent | Analgesic benefit greater than 75% of baseline pain, in lumbar and radicular pain | 4 |
| 2.Very good | Benefit between 50-75% of baseline pain in low back pain and radicular pain. | 5 |
| 3.Good | Benefit greater than 75% in low back pain or radicular pain (One of them) | 4 |
| 4.Moderate | 50-75% benefit in low back pain or radicular pain (One of them) | 1 |
| 5.Poor | Benefit less than 50% in lumbar or radicular pain (One or both). | 0 |

**Table 5: Comparison of the sensitivity thresholds (CPTs) of the groups according to the degree of improvement (excellent VS rest of patients) at three-month follow-up after SCS implant.**

| **Variables** | **Other categories N=10** | **Excellent N=4** | **P-value** |
|---|---|---|---|
| | **Mean (SD)** | **Mean (SD)** | |
| Average | | | |
| Buttock | | | |
| 5 Hz | 0.42 (0.24) | 1.07 (0.49) | 0.01* |
| 250 Hz | 0.54 (0.12) | 1.31 (0.59) | <0.01** |
| 2 kHz | 1.07 (0.30) | 2.18 (0.79) | 0.01* |
| Lumbar | | | |
| 5 Hz | 0.51 (0.25) | 1.87 (1.25) | 0.01* |
| 250 Hz | 0.58 (0.23) | 1.77 (1.08) | 0.01* |
| 2 kHz | 1.52 (0.65) | 2.68 (1.09) | 0.04* |
| Thigh | | | |
| 5 Hz | 0.49 (0.22) | 1.03 (0.86) | 0.11 |
| 250 Hz | 0.51 (0.17) | 0.90 (0.74) | 0.45 |
| 2 kHz | 1.13 (0.23) | 1.93 (1.33) | 0.45 |
| Calf | | | |
| 5 Hz | 0.55 (0.19) | 1.25 (1.00) | 0.02* |
| 250 Hz | 0.68 (0.25) | 1.21 (0.76) | 0.14 |
| 2 kHz | 1.61 (0.54) | 2.26 (1.10) | 0.37 |
| Reference | | | |
| 5 Hz | 0.39 (0.22) | 1.50 (1.31) | 0.05* |
| 250 Hz | 0.38 (0.14) | 1.25 (1.27) | 0.29 |
| 2 kHz | 0.71 (0.30) | 1.60 (1.05) | 0.08 |
| Standardized average | | | |
| Buttock | | | |
| 5 Hz | 1.18 (0.46) | 1.11 (0.77) | 0.54 |
| 250 Hz | 1.51 (0.42) | 1.65 (0.85) | 0.94 |
| 2 kHz | 1.63 (0.50) | 1.58 (0.49) | 0.95 |
| Lumbar | | | |
| 5 Hz | 1.44 (0.56) | 1.60 (0.63) | 0.73 |
| 250 Hz | 1.61 (0.64) | 2.02 (1.02) | 0.37 |
| 2 kHz | 2.24 (0.78) | 1.92 (0.55) | 0.54 |
| Thigh | | | |
| 5 Hz | 1.45 (0.83) | 0.84 (0.34) | 0.19 |
| 250 Hz | 1.39 (0.35) | 1.01 (0.57) | 0.19 |
| 2 kHz | 1.78 (0.59) | 1.21 (0.15) | 0.05* |
| Calf | | | |
| 5 Hz | 1.74 (1.33) | 1.16 (0.68) | 0.60 |
| 250 Hz | 1.90 (0.63) | 1.69 (1.36) | 0.64 |
| 2 kHz | 2.43 (0.64) | 1.72 (0.93) | 0.19 |

**Table 6: Comparison of the sensitivity thresholds (CPTs) of the groups according to the degree of improvement (very good plus excellent vs Other categories) at three-month follow-up after SCS implant.**

| **Variables** | **Other categories N=5 Mean (SD)** | **Very good and excellent N=9 Mean (SD)** | **P-value** |
|---|---|---|---|
| Average | | | |
| Buttock | | | |
| 5 Hz | 0.44(0.34) | 0.70 (0.47) | 0.15 |
| 250 Hz | 0.53(0.14) | 0.88 (0.54) | 0.16 |
| 2 kHz | 1.11(0.27) | 1.55 (0.81) | 0.36 |
| Lumbar | | | |
| 5 Hz | 0.38(0.09) | 1.19 (1.02) | 0.02* |
| 250 Hz | 0.47(0.07) | 1.16 (0.90) | 0.02* |
| 2 kHz | 1.34(0.25) | 2.13 (1.06) | 0.06 |
| Thigh | | | |
| 5 Hz | 0.40(0.23) | 0.78 (0.59) | 0.08 |
| 250 Hz | 0.46(0.17) | 0.71 (0.50) | 0.44 |
| 2 kHz | 1.08(0.25) | 1.51 (0.92) | 0.36 |
| Calf | | | |
| 5 Hz | 0.56(0.31) | 0.86 (0.72) | 0.15 |
| 250 Hz | 0.78(0.26) | 0.86 (0.59) | 0.90 |
| 2 kHz | 1.53(0.54) | 1.95 (0.85) | 0.44 |
| Reference | | | |
| 5 Hz | 0.42(0.27) | 0.87 (1.01) | 0.34 |
| 250 Hz | 0.36(0.12) | 0.78 (0.90) | 0.42 |
| 2 kHz | 0.64(0.24) | 1.15 (0.81) | 0.15 |
| Standardized average | | | |
| Buttock | | | |
| 5 Hz | 1.09(0.47) | 1.20 (0.59) | 1.00 |
| 250 Hz | 1.55(0.37) | 1.56 (0.64) | 0.83 |
| 2 kHz | 1.81(0.38) | 1.50 (0.51) | 0.15 |
| Lumbar | | | |
| 5 Hz | 1.15(0.53) | 1.68 (0.50) | 0.08 |
| 250 Hz | 1.54(0.84) | 1.83 (0.73) | 0.36 |
| 2 kHz | 2.36(1.05) | 2.03 (0.49) | 0.80 |
| Thigh | | | |
| 5 Hz | 1.02(0.36) | 1.42 (0.90) | 0.36 |
| 250 Hz | 1.29(0.09) | 1.28 (0.56) | 0.70 |
| 2 kHz | 1.79(0.45) | 1.52 (0.62) | 0.15 |
| Calf | | | |
| 5 Hz | 1.34(0.52) | 1.66 (1.38) | 0.94 |
| 250 Hz | 2.23(0.42) | 1.62 (0.96) | 0.15 |
| 2 kHz | 2.43(0.49) | 2.11 (0.89) | 0.52 |

### Conclusions

It was therefore found consistent correlations in FBSS patients between average CPTs with irradiated VAS, disability (ODI) and physical health as measured by SF36-PCS meaning that the average CPT can be used to determine whether a FBSS patient is a candidate for SCS surgery. The negative correlation found in physical health is conserved in all the zones evaluated, including the non-affect body-region, and practically at all the frequencies explored. It was also found a steady negative correlation between the CPTs standardized averages and the mental component of the SF36 at 5Hz frequency in all areas studied meaning that standardised average CPT can be used to determine the mental condition of a FBSS patient candidate for SCS surgery. To our knowledge, this is the first time these correlations have been in patients with FBSS.

No differences were found in the degree of hyperesthesia or hypoesthesia manifestations between SC and NSC groups. Patients with hypoesthesia presented higher thresholds than hyperesthesia patients in all the explored territories, including the arm. The main difference in sensitivity between NSC and SC groups was seen in the upper limb, with lower thresholds in the NSC group, although not reaching statistical significance. The NSC group, at 5 Hz frequency, showed a not statistically significant trend to have lower average thresholds than SC group in the reference region, and higher standardized average in the lumbar and buttock regions. These results, together with the correlation of the standardized mean of CPTs at 5Hz with SF36-MCS (i.e. the greater the difference between the pathological zone explored and the reference zone, the worse is the mental health), in all the explored regions, suggests that the lowering of thresholds may be caused by affective disorders.

The significant higher CPTs in patients with a better response to SCS, could represent they have a more efficient somatosensory system, both at spinal and supraspinal level.

The significant correlations found in the study are in all cases above the recommended threshold (r ≥ 0.30) to be clinically relevant in patient-reported outcomes measures in chronic disease. In conclusion, significant correlations have been established between CPTs and the physical and mental health of patients with FBSS meaning that the CPT can be used to classify FBSS patients eligible for SCS surgery. In addition, the CPTs in FBSS patients can assist to select the best SCS candidates.

The study shows that the standardised and/or non-standardised average sensitivity thresholds for the buttock, lumbar, thigh, and calf areas correlate to the outcome of the patient for different stimulation frequencies which stimulate different fibre-types. Significant correlations (absolute value greater than 0.3 with a low p-value) are found for C-type, Aδ-type and/or Aβ-type fibre stimulation and for the buttock, lumbar, thigh and/or calf. This is a strong indicator that the standardised and/or the non-standardised threshold values may be reliable source of information to classify patients with failed back surgery syndrome (FBSS) eligible for spinal cord stimulation (SCS) treatment related to the C-type, Aδ-type and/or Aβ-type fibres. (In the case of the standardised average thresholds, there is observed a correlation with the mental health of the patient which may be a determinative factor in choosing candidates for surgery).

### CLAUSES

1. A system for Current Perception Threshold (CPT) quantification of a user, the system configured to generate data for classifying patients with failed back surgery syndrome (FBSS) eligible for spinal cord stimulation (SCS) treatment, wherein the system comprises:
   a) one or more electrode pairs;
   b) a stimulator configured to provide a plurality of electrical stimuli between the pair of electrodes of each of the one or more electrode pairs, wherein the plurality of electrical stimuli comprises a plurality of AC signals of a frequency from a predefined set of frequencies;
   c) a feedback element configured to determine whether the electrical stimuli is felt by the user; and
   d) a processor, wherein the processor is configured to control the stimulator and receive an indication from the feedback element whether the electrical stimuli is felt by the user;
      wherein the one or more electrode pairs are provided on a substrate for placing the electrode pairs to the skin of the user, wherein the system is further configured to trigger the electrical stimuli of the buttock, the lumbar, the thigh and/or the calf skin areas of the user through the one or more of electrode pairs, wherein each electrode pair defines a sub-area within the area;
      wherein the processor is further configured to:
         i. for each frequency of the set of frequencies for each of a plurality of electrode pair positions, to receive an indication of the location of the electrode pair positions on the buttock, the lumbar, the thigh and/or the calf, and control the stimulator to provide two or more electrical stimuli at the frequency and electrode pair positions, the two or more electrical stimuli having different electrical intensities;
         ii. for each electrical stimulus, receive an indication by the feedback element whether the electrical stimuli was felt by the user;
         iii. for each frequency at each electrode pair position, determine a threshold value for the lowest electrical intensity felt by the user based on the received indications for each electrical stimulus; and
         iv. for each frequency for the buttock, the lumbar, the thigh and/or the calf, calculate an average threshold value indicative of the lowest electrical intensity felt by the user, based at least in part on the threshold values determined in iii).
2. The system according to clause 1, wherein the system is further configured for classifying patients with failed back surgery syndrome (FBSS) by mental condition and the average threshold values obtained in iv) include standardised threshold values, wherein the processor is further configured to:
   v. for each frequency of the plurality of frequencies for a non-FBSS-affected electrode pair position to activate the stimulator to provide two or more electrical stimuli at the frequency and non-FBSS-affected electrode pair position, the two or more electrical stimuli having different electrical intensities;
   vi. for each electrical stimulus at the non-FBSS-affected electrode pair position, determine by the feedback element whether the electrical stimuli were felt by the user;
   vii. for each frequency at the non-FBSS-affected electrode pair position, determine a non-FBSS threshold value for the lowest electrical intensity felt by the user based on the received indications for each electrical stimulus; and
   viii. calculate a standardised average threshold value indicative of the difference between the average of the threshold values calculated in iii) and the corresponding non-FBSS threshold value for one or more frequencies at one or more electrode pair positions.
3. The system according to any of the previous clauses, wherein the processor is further configured to:
   ix. receive an indication of the location of an electrode pair position and control the stimulator to provide one or more preliminary stimuli to the user at the electrode pair position;
   x. receive one or more indications via the feedback element whether the electrical stimuli were felt by the user; and
   xi. determine a reference range of electrical intensity based in the received indications.
4. The system according to any of the previous clauses, wherein the processor is further configured to:
   xii. for one or more frequencies and for a reference electrode pair position to receive an indication of the location of the reference electrode pair position on the buttock, the lumbar, the thigh and/or the calf, and control the stimulator to provide two or more electrical stimuli at the frequency and reference electrode pair position, the two or more electrical stimuli having different electrical intensities;
   xiii. for each electrical stimulus at the reference electrode pair position, receive an indication via the feedback element whether the electrical stimuli were felt by the user;
   xiv. for each frequency at the reference electrode pair position, determine a reference range of electrical intensity based on the received indications for each electrical stimulus;
      wherein one or more of the electrical stimuli of i) have an intensity within the reference range.
5. The system according to clause 4, wherein for each frequency at the reference electrode pair position, xii) comprises providing a first plurality of stimuli sequentially ascending in electrical intensity, and/or a second plurality of stimuli sequentially descending in electrical intensity; optionally wherein the reference range of electrical intensity is determined based on the received indications in xiii) by selecting as maximum and minimum for the reference range the highest and lowest electrical intensities wherein a change in the received indications is noted.
6. The system according to clause 4 or 5, wherein if the difference between the limits of the reference range of electrical intensities is bigger than a predetermined value v1, the processor is configured to repeat the steps xii-xiv) until the reference range is less than v1, unless a predetermined maximum number of iterations (iter) is reached, preferably wherein the electrical intensity is the electrical current intensity and v1 is 0.5mA and preferably wherein iter is equal or bigger than three.
7. The system according to any of the previous clauses, wherein the threshold value of step iii) is determined by:
   - selecting the lowest electrical intensity indicated as being felt by the user; or
   - selecting the lowest electrical intensity indicated as being felt by the user wherein the immediately higher electrical intensity is also indicated as being felt by the user; or
   - selecting the lowest electrical intensity indicated as being felt by the user wherein the threshold value for the lowest electrical intensity is indicated as felt by the user at least twice; or
   - selecting the lowest electrical intensity indicated as being felt by the user wherein the threshold value for the lowest electrical intensity is indicated as felt by the user more than half of the times; or
   - selecting the lowest electrical intensity indicated as being felt by the user wherein the threshold value for the lowest electrical intensity is indicated as felt by the user all the times.
8. The system according to any of the previous clauses, wherein if all of the electrical stimuli of step i) are indicated as being felt by the user, steps i) and ii) are repeated for a second plurality of electrical stimuli of lower intensities, and/or wherein if none of the electrical stimuli of step i) are indicated as being felt by the user, steps i) and ii) are repeated for a second plurality of electrical stimuli of higher intensities.
9. The system according to any of the previous clauses, wherein the system further comprises a memory and the processor is configured to store, for each of the electrical stimuli, data indicating one or more of the following:
   - the intensity, frequency, electrode pair position, the location of the electrode pair position on the buttock, the lumbar, the thigh and/or the calf of i) and/or the location of the electrode pair position of the non-FBSS-affected area of v);
   - whether the electrical stimulus has been felt by the user of ii) and/or vi);
   - the threshold value of iii) and/or vii); and
   - the average threshold value of iv), optionally wherein the average threshold value is the standardised average threshold value of viii).
10. A system for Current Perception Threshold (CPT) quantification of a user configured to prognosticate the outcome of a patient with failed back surgery syndrome (FBSS) undergoing a spinal cord stimulation (SCS) treatment, wherein the system is any of clauses 1 to 9 and the processor is further configured to:
   - compare the one or more average threshold values with one or more predetermined standard average threshold values; and
   - determine the outcome of the patient undergoing an SCS treatment from the significant deviations found in the comparison.
11. The system according to any of the previous clauses, wherein the predetermined set of frequencies comprises:
   - a first frequency for preferentially stimulating the C-type fibres; and/or
   - a second frequency for preferentially stimulating the Aδ-type fibres; and/or
   - a third frequency for preferentially stimulating the Aβ-type fibres.
12. The system according to any of the previous clauses, wherein the processor is further configured to generate a set of data from the determined CPT wherein the data indicates one or more of the following:
   - the threshold value and/or average threshold value as a function of stimuli frequency for the plurality of electrode pair positions; and
   - one or more maps of the threshold value as a function of electrode pair position, each map indicating the threshold value for a single frequency.
13. The system according to clause 12, wherein the system further comprises a display device configured to display the set of data, wherein the processor is further configured to display the set of data on the display device.
14. A computer-implemented method to generate data for classifying patients with failed back surgery syndrome (FBSS) eligible for spinal cord stimulation (SCS) treatment, wherein the method comprises the steps:
   a) receiving data obtained from one or more electrode pairs placed at a plurality of electrode pair positions on a user, wherein for each frequency of a plurality of frequencies at each electrode pair position, two or more electrical stimuli at the frequency and having different electrical intensities were provided, wherein the data comprises, for each electrical stimuli and electrode pair position: the location of the electrode pair position on the buttock, the lumbar, the thigh and/or the calf; the frequency of the electrical stimulus, the electrical intensity of the electrical stimulus and an indication of whether the electrical stimulus was felt by the user;
   b) for each frequency for the buttock, the lumbar, the thigh and/or the calf, determining a threshold value for the lowest electrical intensity sensitive to the user based on the received indication for each electrical stimulus; and
   c) for each frequency for the buttock, the lumbar, the thigh and/or the calf, calculating an average threshold value indicative of the lowest electrical intensity felt by the user, based at least in part the threshold values determined in b).
15. The method according to clause 14, wherein the method further comprises the steps of:
   d) receiving data indicating a non-FBSS threshold value for the lowest electrical intensity felt by the user in a non-FBSS-affected area; and
   e) generating data for classifying patients with FBSS eligible for SCS treatment from the determined CPT by calculating a standardised average threshold value indicative of the difference between the average of the threshold values of b) and the corresponding non-FBSS threshold value.
16. The method according to clauses 14 or 15, wherein the method further comprises the step of:
   f) generating data for classifying patients with FBSS eligible for SCS treatment from the determined CPT wherein the data indicates one or more of the following:
   - the threshold value as a function of stimuli frequency for the plurality of electrode pair positions; and
   - one or more maps of the threshold value as a function of electrode pair position, each map indicating the threshold value for a single frequency.
17. A computer-implemented method to prognosticate the outcome of a patient with failed back surgery syndrome (FBSS) undergoing a spinal cord stimulation (SCS) treatment, wherein the method comprises the steps of any of the clauses 14 to 16 and further comprises the steps:
   g) comparing the generated data with one or more standard values for the data; and
   h) determining the outcome of the patient undergoing an SCS treatment from the significant deviations found in the comparison.
18. A computer program product comprising instructions which, when the program is executed by a processor, cause the computer to carry out the steps of the method of any of the clauses 14 to 17.

## Claims

1. A system for Current Perception Threshold (CPT) quantification of a user, the system configured to generate data for classifying patients with failed back surgery syndrome (FBSS) eligible for spinal cord stimulation (SCS) treatment, wherein the system comprises:
e) one or more electrode pairs;
f) a stimulator configured to provide a plurality of electrical stimuli between the pair of electrodes of each of the one or more electrode pairs, wherein the plurality of electrical stimuli comprises a plurality of AC signals of a frequency from a predefined set of frequencies;
g) a feedback element configured to determine whether the electrical stimuli is felt by the user; and
h) a processor, wherein the processor is configured to control the stimulator and receive an indication from the feedback element whether the electrical stimuli is felt by the user;
wherein the one or more electrode pairs are provided on a substrate for placing the electrode pairs to the skin of the user, wherein the system is further configured to trigger the electrical stimuli of the buttock, the lumbar, the thigh and/or the calf skin areas of the user through the one or more of electrode pairs, wherein each electrode pair defines a sub-area within the area;
wherein the processor is further configured to:
v. for each frequency of the set of frequencies for each of a plurality of electrode pair positions, to receive an indication of the location of the electrode pair positions on the buttock, the lumbar, the thigh and/or the calf, and control the stimulator to provide two or more electrical stimuli at the frequency and electrode pair positions, the two or more electrical stimuli having different electrical intensities;
vi. for each electrical stimulus, receive an indication by the feedback element whether the electrical stimuli was felt by the user;
vii. for each frequency at each electrode pair position, determine a threshold value for the lowest electrical intensity felt by the user based on the received indications for each electrical stimulus; and
viii. for each frequency for the buttock, the lumbar, the thigh and/or the calf, calculate an average threshold value indicative of the lowest electrical intensity felt by the user, based at least in part on the threshold values determined in iii).

2. The system according to claim 1, wherein the system is further configured for classifying patients with failed back surgery syndrome (FBSS) by mental condition and the average threshold values obtained in iv) include standardised threshold values, wherein the processor is further configured to:
ix. for each frequency of the plurality of frequencies for a non-FBSS-affected electrode pair position to activate the stimulator to provide two or more electrical stimuli at the frequency and non-FBSS-affected electrode pair position, the two or more electrical stimuli having different electrical intensities;
x. for each electrical stimulus at the non-FBSS-affected electrode pair position, determine by the feedback element whether the electrical stimuli were felt by the user;
xi. for each frequency at the non-FBSS-affected electrode pair position, determine a non-FBSS threshold value for the lowest electrical intensity felt by the user based on the received indications for each electrical stimulus; and
xii. calculate a standardised average threshold value indicative of the difference between the average of the threshold values calculated in iii) and the corresponding non-FBSS threshold value for one or more frequencies at one or more electrode pair positions.

3. The system according to any of the previous claims, wherein the processor is further configured to:
xiii. receive an indication of the location of an electrode pair position and control the stimulator to provide one or more preliminary stimuli to the user at the electrode pair position;
xiv. receive one or more indications via the feedback element whether the electrical stimuli were felt by the user; and
xv. determine a reference range of electrical intensity based in the received indications.

4. The system according to any of the previous claims, wherein the threshold value of step iii) is determined by:
- selecting the lowest electrical intensity indicated as being felt by the user; or
- selecting the lowest electrical intensity indicated as being felt by the user wherein the immediately higher electrical intensity is also indicated as being felt by the user; or
- selecting the lowest electrical intensity indicated as being felt by the user wherein the threshold value for the lowest electrical intensity is indicated as felt by the user at least twice; or
- selecting the lowest electrical intensity indicated as being felt by the user wherein the threshold value for the lowest electrical intensity is indicated as felt by the user more than half of the times; or
- selecting the lowest electrical intensity indicated as being felt by the user wherein the threshold value for the lowest electrical intensity is indicated as felt by the user all the times.

5. The system according to any of the previous claims, wherein if all of the electrical stimuli of step i) are indicated as being felt by the user, steps i) and ii) are repeated for a second plurality of electrical stimuli of lower intensities, and/or wherein if none of the electrical stimuli of step i) are indicated as being felt by the user, steps i) and ii) are repeated for a second plurality of electrical stimuli of higher intensities.

6. The system according to any of the previous claims, wherein the system further comprises a memory and the processor is configured to store, for each of the electrical stimuli, data indicating one or more of the following:
- the intensity, frequency, electrode pair position, the location of the electrode pair position on the buttock, the lumbar, the thigh and/or the calf of i) and/or the location of the electrode pair position of the non-FBSS-affected area of v);
- whether the electrical stimulus has been felt by the user of ii) and/or vi);
- the threshold value of iii) and/or vii); and
- the average threshold value of iv), optionally wherein the average threshold value is the standardised average threshold value of viii).

7. A system for Current Perception Threshold (CPT) quantification of a user configured to prognosticate the outcome of a patient with failed back surgery syndrome (FBSS) undergoing a spinal cord stimulation (SCS) treatment, wherein the system is any of claims 1 to 6 and the processor is further configured to:
- compare the one or more average threshold values with one or more predetermined standard average threshold values; and
- determine the outcome of the patient undergoing an SCS treatment from the significant deviations found in the comparison.

8. The system according to any of the previous claims, wherein the predetermined set of frequencies comprises:
- a first frequency for preferentially stimulating the C-type fibres; and/or
- a second frequency for preferentially stimulating the Aδ-type fibres; and/or
- a third frequency for preferentially stimulating the Aβ-type fibres.

9. The system according to any of the previous claims, wherein the processor is further configured to generate a set of data from the determined CPT wherein the data indicates one or more of the following:
- the threshold value and/or average threshold value as a function of stimuli frequency for the plurality of electrode pair positions; and
- one or more maps of the threshold value as a function of electrode pair position, each map indicating the threshold value for a single frequency.

10. The system according to claim 9, wherein the system further comprises a display device configured to display the set of data, wherein the processor is further configured to display the set of data on the display device.

11. A computer-implemented method to generate data for classifying patients with failed back surgery syndrome (FBSS) eligible for spinal cord stimulation (SCS) treatment, wherein the method comprises the steps:
d) receiving data obtained from one or more electrode pairs placed at a plurality of electrode pair positions on a user, wherein for each frequency of a plurality of frequencies at each electrode pair position, two or more electrical stimuli at the frequency and having different electrical intensities were provided, wherein the data comprises, for each electrical stimuli and electrode pair position: the location of the electrode pair position on the buttock, the lumbar, the thigh and/or the calf; the frequency of the electrical stimulus, the electrical intensity of the electrical stimulus and an indication of whether the electrical stimulus was felt by the user;
e) for each frequency for the buttock, the lumbar, the thigh and/or the calf, determining a threshold value for the lowest electrical intensity sensitive to the user based on the received indication for each electrical stimulus; and
f) for each frequency for the buttock, the lumbar, the thigh and/or the calf, calculating an average threshold value indicative of the lowest electrical intensity felt by the user, based at least in part the threshold values determined in b).

12. The method according to claim 11, wherein the method further comprises the steps of:
g) receiving data indicating a non-FBSS threshold value for the lowest electrical intensity felt by the user in a non-FBSS-affected area; and
h) generating data for classifying patients with FBSS eligible for SCS treatment from the determined CPT by calculating a standardised average threshold value indicative of the difference between the average of the threshold values of b) and the corresponding non-FBSS threshold value.

13. The method according to claims 11 or 12, wherein the method further comprises the step of:
i) generating data for classifying patients with FBSS eligible for SCS treatment from the determined CPT wherein the data indicates one or more of the following:
- the threshold value as a function of stimuli frequency for the plurality of electrode pair positions; and
- one or more maps of the threshold value as a function of electrode pair position, each map indicating the threshold value for a single frequency.

14. A computer-implemented method to prognosticate the outcome of a patient with failed back surgery syndrome (FBSS) undergoing a spinal cord stimulation (SCS) treatment, wherein the method comprises the steps of any of the claims 11 to 13 and further comprises the steps:
j) comparing the generated data with one or more standard values for the data; and
k) determining the outcome of the patient undergoing an SCS treatment from the significant deviations found in the comparison.

15. A computer program product comprising instructions which, when the program is executed by a processor, cause the computer to carry out the steps of the method of any of the claims 11 to 14.
